(19) Europäisches Patentamt
European Patent Office
Office européen des brevets

(11) **EP 1 855 677 B1**

(12) **EUROPEAN PATENT SPECIFICATION**

(45) Date of publication and mention of the grant of the patent:
**03.12.2008 Bulletin 2008/49**

(21) Application number: **06765238.8**

(22) Date of filing: **04.08.2006**

(51) Int Cl.:
***A61K 31/473*** *(2006.01)* ***A61P 25/18*** *(2006.01)*

(86) International application number:
**PCT/GB2006/002936**

(87) International publication number:
**WO 2007/017654 (15.02.2007 Gazette 2007/07)**

(54) **3,11B CIS DIHYDROTETRABANEZINE FOR THE TREATMENT OF SCHIZOPHRENIA AND OTHER PSYCHOSES**

3,11B-CIS-DIHYDROTETRABANEZIN ZUR BEHANDLUNG VON SCHIZOPHRENIE UND ANDEREN PSYCHOSEN

3,11B CIS-DIHYDROTETRABENAZINE PERMETTANT DE TRAITER LA SCHIZOPHRENIE ET D'AUTRES PSYCHOSES

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(30) Priority: **06.08.2005 GB 0516167**
**16.08.2005 GB 0516790**

(43) Date of publication of application:
**21.11.2007 Bulletin 2007/47**

(73) Proprietor: **Cambridge Laboratories (Ireland) Limited**
**Dublin 4 (IE)**

(72) Inventors:
- **DUFFIELD, Andrew, John**
**Tyne and Wear NE28 9NX (GB)**
- **YARROW, Jean Elisabeth**
**London SW2 5BZ (GB)**

(74) Representative: **Hutchins, Michael Richard**
**Elkington and Fife LLP**
**Prospect House**
**8 Pembroke Road**
**Sevenoaks**
**Kent TN13 1XR (GB)**

(56) References cited:
**WO-A-20/05077946**

- **MEHVAR R ET AL: "CONCENTRATION-EFFECT RELATIONSHIPS OF TETRABENAZINE AND DIHYDROTETRABENAZINE IN THE RAT" JOURNAL OF PHARMACEUTICAL SCIENCES, vol. 76, no. 6, 1987, pages 461-465, XP009075267 ISSN: 0022-3549**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

This invention relates to the use of a dihydrotetrabenazine for the manufacture of a medicament for the prophylaxis or treatment of a psychosis.

## Background of the Invention

Psychosis is a generic psychiatric term for mental states in which the components of rational thought and perception are severely impaired. Persons experiencing a psychosis may experience hallucinations, hold paranoid or delusional beliefs, demonstrate personality changes and exhibit disorganized thinking. This is usually accompanied by a lack of insight into the unusual or bizarre nature of their behavior, difficulties with social interaction and impairments in carrying out the activities of daily living. Essentially, a psychotic episode involves loss of contact with reality.

Psychosis is often considered to be a symptom of severe mental illness. Although it is not exclusively linked to any particular psychological or physical state, it is particularly associated with schizophrenia, bipolar disorder (manic depression) and severe clinical depression. There are also several physical circumstances that can induce a psychotic state, including electrolyte disorder, urinary tract infections in the elderly, pain syndromes, drug toxicity, and drug withdrawal (especially alcohol, barbiturates, and sometimes benzodiazepines), as well as infections of or injuries to the brain (these psychoses are now more commonly referred to as organic mental disorders).

Psychosis may be caused by or follow brain injury and may occur after drug use, particularly after drug overdose, chronic use, and during drug withdrawal.

Chronic psychological stress is also known to cause psychotic states, although the exact mechanism by which this occurs is uncertain. Short-lived psychosis triggered by stress is known as brief reactive psychosis.

Psychotic episodes can be significantly coloured by mood. For example, people experiencing a psychotic episode in the context of depression may experience persecutory or self-blaming delusions or hallucinations, whilst people experiencing a psychotic episode in the context of mania may form grandiose delusions or have an experience of deep religious significance.

Hallucinations are defined as sensory perception in the absence of external stimuli. Psychotic hallucinations may occur in any of the five senses and take on almost any form, which may include simple sensations (such as lights, colours, tastes, smells) to more meaningful experiences such as seeing and interacting with fully formed animals and people, hearing voices and complex tactile sensations.

Auditory hallucinations, particularly the experience of hearing voices, are a common and often prominent feature of psychosis. Hallucinated voices may talk about, or to, the person, and may involve several speakers with distinct personas. Auditory hallucinations tend to be particularly distressing when they are derogatory, commanding or preoccupying.

Psychosis may involve delusional or paranoid beliefs. Psychotic delusions can be classified into primary and secondary types. Primary delusions are defined as arising out-of-the-blue and not being comprehensible in terms of normal mental processes, whereas secondary delusions may be understood as being influenced by the person's background or current situation.

Thought disorder describes an underlying disturbance to conscious thought and is classified largely by its effects on speech and writing. Affected persons may show pressure of speech (speaking incessantly and quickly), derailment or flight of ideas (switching topic mid-sentence or inappropriately), thought blocking, rhyming or punning.

One important and poorly understood feature of psychosis is usually an accompanying lack of insight into the unusual, strange or bizarre nature of the person's experience or behaviour. Even in the case of an acute psychosis, sufferers may seem completely unaware that their vivid hallucinations and impossible delusions are in any way unrealistic. However, insight can vary between individuals and throughout the duration of the psychotic episode. In some cases, particularly with auditory and visual hallucinations, the patient has good insight and this makes the psychotic experience even more terrifying in that the patient realizes that he or she should not be hearing voices, but does.

There are a number of possible causes for psychosis. Psychosis may be the result of an underlying mental illness such as Bipolar disorder (also known as manic depression), and schizophrenia. Psychosis may also be triggered or exacerbated by severe mental stress and high doses or chronic use of drugs such as amphetamines, LSD, PCP, cocaine or scopolamine. Sudden withdrawal from CNS depressant drugs, such as alcohol and benzodiazepines, may also trigger psychotic episodes. As can be seen from the wide variety of illnesses and conditions in which psychosis has been reported to arise (including for example, AIDS, leprosy, malaria and even mumps) there is no singular cause of a psychotic episode.

Schizophrenia is the name given to a group of psychotic disorders usually characterized by withdrawal from reality, illogical patterns of thinking, delusions, and hallucinations, and accompanied in varying degrees by other emotional, behavioral, or intellectual disturbances. Schizophrenia is associated with dopamine imbalances in the brain and defects of the frontal lobe and is caused by genetic and other biological factors and psychosocial factors.

The drugs traditionally used to treat psychoses such as those associated with schizophrenia (the so-called "typical" antipsychotics) effectively control the hallucinations, delusions, and confusion associated with these conditions. Such drugs, examples of which include haloperidol, chlorpromazine, and fluphenazine, have been available since the mid-1950s. These drugs act primarily by blocking dopamine receptors and are effective in treating the "positive" symptoms of psychosis.

Four major areas of the brain are involved as primary pathways for dopamine. They include the nigrostriatal, mesocortical, mesolimbic, and tuberoinfundibular systems. Decreased dopamine activity in the mesocortical tract (as seen in the schizophrenic patient) results in an inability for the prefrontal areas of the brain to activate. Positive symptoms, such as hallucinations and delusions, can occur when overactivity of dopamine in the mesolimbic tract occurs. There are five subcategories of dopamine receptors in the brain. Conventional antipsychotics have the greatest impact on the D2 receptor. The so-called "atypical" antipsychotic agents (see below) typically have a weaker effect on D2 receptors with more potent blockade on the D4 receptor which is mostly found in the frontal cortex and the hippocampus.

Conventional ("typical") antipsychotics block D2 receptors nonselectively in all four areas of the brain. The resulting effect in the mesolimbic tract reduces hallucinations and delusions. However, a concurrent reduction in dopamine in the nigrostriatal pathway can produce extrapyramidal symptoms. Blockade of dopamine may also worsen negative symptoms and cognitive functioning by further decreasing the amount of dopamine in the frontal cortex. The tuberoinfundibular tract is affected by all the conventional antipsychotics, which may cause neuroendocrine and hypothalamic dysfunction. Dopamine blockage in the tuberoinfundibular tract is responsible for increases in prolactin levels.

Thus, the use of "typical" anti-psychotics is associated with a number of undesirable side effects.

The atypical antipsychotics target the limbic area more specifically when blocking dopamine D2 receptors. Consequently, they have less impact on the nigrostriatal and mesocortical pathways, resulting in a reduced potential for adverse effects. As noted earlier, they also tend to have a greater affinity for dopamine D4 receptors.

The receptor binding profiles of atypical antipsychotic drugs is reviewed in the article by A. E. Hensiek & M. R. Trimble, J Neurology, Neurosurgery and Psychiatry, (2002), 72:281-285.

The newer "atypical" antipsychotics - often referred to as the serotonin-dopamine antagonists (SDAs) - block both serotonin and dopamine receptors, thereby treating both the "positive" and "negative" symptoms of schizophrenia - see H. Y. Meltzer, J. Clin. Psychopharmacol. (1995), Feb;15(1 Suppl 1):2S-3S and M. Huttunen,. J. Clin. Psychopharmacol. (1995), Feb;15(1 Suppl 1):4S-10S. These newer medications are effective in treating a broader range of symptoms of psychosis and schizophrenia, and have fewer side effects than traditional antipsychotics. For example, they have a lower propensity than typical antipsychotics to cause extrapyramidal side effects and prolactin elevation.

Examples of these newer atypical antipsychotics (the "serotonin-dopamine antagonists") include clopazine, risperidone, asenapine, olanzapine and iloperidone.

Tetrabenazine (Chemical name: 1, 3, 4,6,7,11b-hexahydro-9,10-dimethoxy-3-(2-methylpropyl)-2H-benzo(a) quinolizin-2-one) has been in use as a pharmaceutical drug since the late 1950s. Initially developed as an anti-psychotic, tetrabenazine is currently used in the symptomatic treatment of hyperkinetic movement disorders such as Huntington's disease, hemiballismus, senile chorea, tic, tardive dyskinesia and Tourette's syndrome, see for example Jankovic et al., Am. J Psychiatry. (1999) Aug; 156(8):1279-81 and Jankovic et al., Neurology (1997) Feb; 48(2):358-62.

The chemical structure of tetrabenazine is as shown in Figure 1 below.

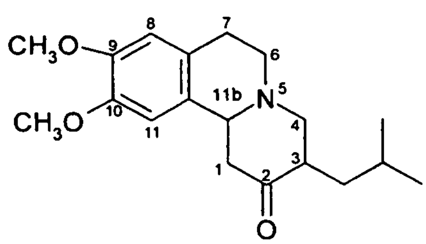

Figure 1- Structure of tetrabenazine

The compound has chiral centres at the 3 and 11 b carbon atoms and hence can, theoretically, exist in a total of four isomeric forms, as shown in Figure 2.

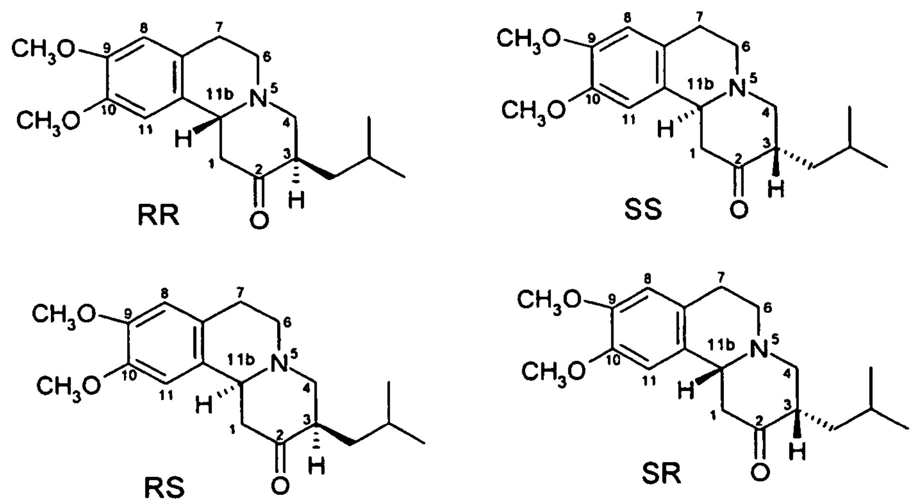

RR SS RS SR

Figure 2 – Possible tetrabenazine isomers

In Figure 2, the stereochemistry of each isomer is defined using the "R and S" nomenclature developed by Cahn, Ingold and Prelog, see Advanced Organic Chemistry by Jerry March, 4th Edition, John Wiley & Sons, New York, 1992, pages 109-114. In Figure 2 and elsewhere in this patent application, the designations "R" or "S" are given in the order of the position numbers of the carbon atoms. Thus, for example, *RS* is a shorthand notation for 3*R,* 11b*S*. Similarly, when three chiral centres are present, as in the dihydrotetrabenazines described below, the designations "*R*" or "*S*" are listed in the order of the carbon atoms 2, 3 and 11b. Thus, the 2*S*,3*R*,*11bR* isomer is referred to in short hand form as *SRR* and so on.

Commercially available tetrabenazine is a racemic mixture of the *RR* and *SS* isomers and it would appear that the *RR* and *SS* isomers (hereinafter referred to individually or collectively as *trans*-tetrabenazine because the hydrogen atoms at the 3 and 11b positions have a *trans* relative orientation) are the most thermodynamically stable isomers.

Tetrabenazine has somewhat poor and variable bioavailability. It is extensively metabolised by first-pass metabolism, and little or no unchanged tetrabenazine is typically detected in the urine. The major metabolite is dihydrotetrabenazine (Chemical name: 2-hydroxy-3-(2-methylpropyl)-1,3,4,6,7,11b-hexahydro-9,10-dimethoxy-benzo(a) quinolizine) which is formed by reduction of the 2-keto group in tetrabenazine, and is believed to be primarily responsible for the activity of the drug (see Mehvar et al., Drug Metab.Disp, 15, 250-255 (1987) and J Pharm. Sci., 76, No.6, 461-465 (1987)).

Four dihydrotetrabenazine isomers have previously been identified and characterised which are derived from the more stable *RR* and *SS* isomers of the parent tetrabenazine and have a *trans* relative orientation between the hydrogen atoms at the 3 and 11b positions) (see Kilbourn et al., Chirality, 9:59-62 (1997) and Brossi et al., Helv. Chim. Acta., vol. XLI, No. 193, pp1793-1806 (1958). The four isomers are (+)-α-dihydrotetrabenazine, (-)-α-dihydrotetrabenazine, (+)-β-dihydrotetrabenazine and (-)-β-dihydrotetrabenazine. The structures of the four known *trans*-dihydrotetrabenazine isomers are considered to be as shown in Figure 3.

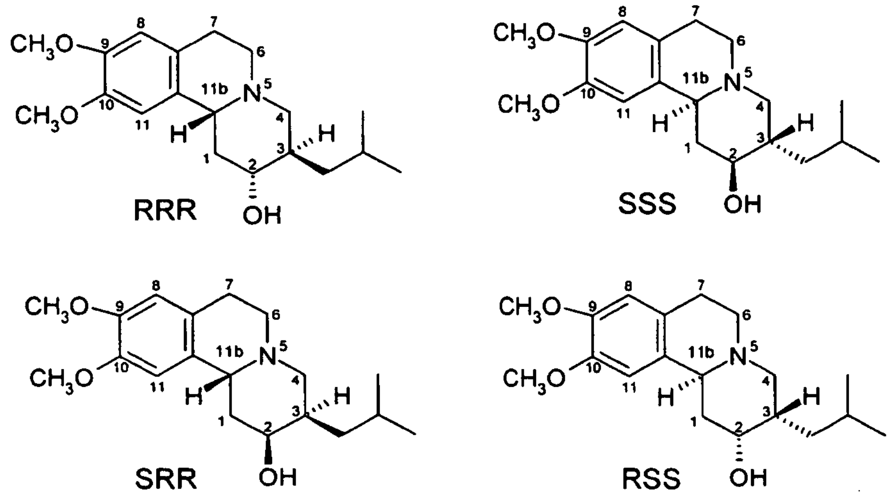

RRR SSS SRR RSS

Figure 3 – Structures of known isomers of dihydrotetrabenazine

Kilbourn et al., (see Eur. J Pharmacol., 278:249-252 (1995) and Med. Chem. Res., 5:113-126 (1994)) investigated the specific binding of individual radio-labelled dihydrotetrabenazine isomers in the conscious rat brain. They found that the (+)-α-[$^{11}$C]dihydrotetrabenazine (2*R*,3*R*,11b*R*) isomer accumulated in regions of the brain associated with higher concentrations of the neuronal membrane dopamine transporter (DAT) and the vesicular monoamine transporter (VMAT2). However, the essentially inactive (-)-α-[$^{11}$C]dihydrotetrabenazine isomer was almost uniformly distributed in the brain, suggesting that specific binding to DAT and VMAT2 was not occurring. The *in vivo* studies correlated with *in vitro* studies which demonstrated that the (+)-α-[$^{11}$C]dihydrotetrabenazine isomer exhibits a $K_i$ for [$^3$H]methoxytetrabenazine >2000-fold higher than the $K_i$ for the (-)-α-[$^{11}$C]dihydrotetrabenazine isomer.

Our earlier International patent application No. PCT/GB2005/000464 discloses the preparation and use of pharmaceutical dihydrotetrabenazine isomers derived from the unstable *RS* and *SR* isomers (hereinafter referred to individually or collectively as cis-tetrabenazine because the hydrogen atoms at the 3 and 11b positions have a *cis* relative orientation) of tetrabenazine.

There are four possible isomers of dihydrotetrabenazine having the 3,11 b-*cis* configuration and these are the 2*S*,3*S*,11b*R* isomer, the 2*R*,3*R*,11b*S* isomer, the 2*R*,3*S*,11b*R* isomer and the 2*S*,3*R*, 11b*S* isomer, which are as follows:

(a) the 2*S*,3*S*,11b*R* isomer of 3,11b-*cis*-dihydrotetrabenazine having the formula (Ia):

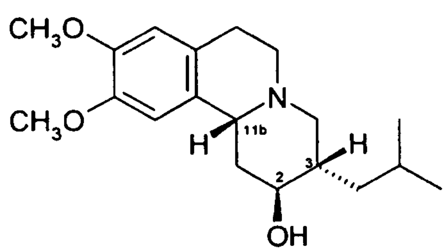

(Ia)

(b) the 2*R*,3*R*,11b*S* isomer of 3,11b-*cis*-dihydrotetrabenazine having the formula (Ib):

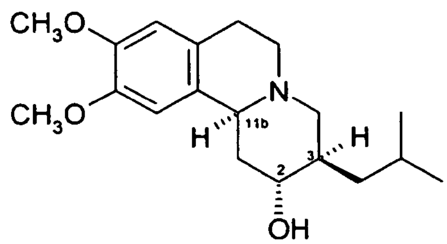

(Ib)

(c) the 2*R*,3*S*,11b*R* isomer of 3,11b-*cis*-dihydrotetrabenazine having the formula (Ic):

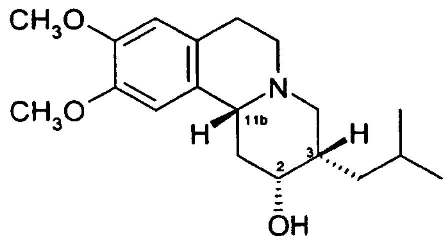

(Ic)

and

(d) the 2*S*,3*R*,11b*S* isomer of 3,11b-*cis*-dihydrotetrabenazine having the formula (Id):

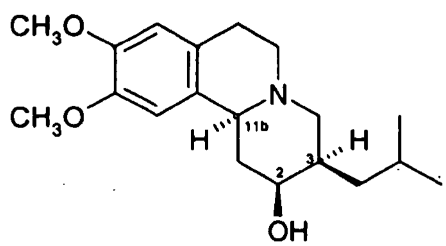

## Summary of the Invention

It has now been found that the 2*R*,3*R*,11b*S* isomer of 3,11b-*cis*-dihydrotetrabenazine of the formula (Ib) described in our earlier application no. PCT/GB2005/000464 demonstrates receptor binding profiles broadly similar to the receptor binding profiles of atypical antipsychotic agents. In particular, the 2*R*,3*R*, 11b*S* isomer exhibits both dopaminergic and serotinergic inhibitory actions. The receptor binding profile indicates that the 2*R*,3*R*, 11b*S* isomer will be of use in the prophylaxis or treatment of psychosis, for example psychosis arising from or associated with schizophrenia,

Accordingly, in a first aspect, the invention provides a 3, 11b-*cis*-dihydrotetrabenazine of the formula (Ib):

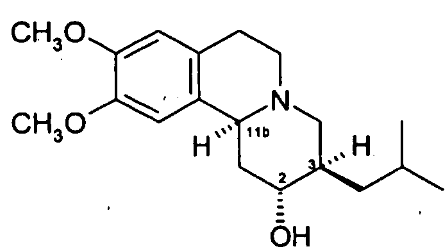

(Ib)

or a pharmaceutically acceptable salt thereof for use in the prophylaxis or treatment of psychosis.

In another aspect, the invention provides a 3,11b-*cis*-dihydrotetrabenazine of the formula (Ib):

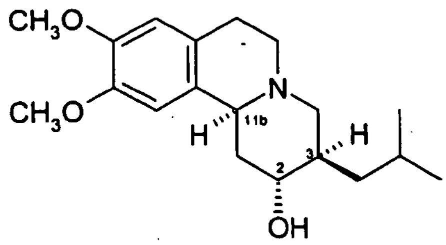

(Ib)

or a pharmaceutically acceptable salt thereof for use in the prophylaxis or treatment of schizophrenia.

The compound of formula (Ib), the 2*R*,3*R*,11b*S* isomer of 3,11b-*cis*- dihydrotetrabenazine, is also referred to herein as Isomer A.

The invention also provides:

- The use of 3, 11b-*cis*-dihydrotetrabenazine Isomer A for the manufacture of a medicament for (i) the prophylaxis or treatment of psychosis; or (ii) the prophylaxis or treatment of schizophrenia.
- The use of 3, 11b-*cis*-dihydrotetrabenazine Isomer A for the manufacture of a medicament for preventing or alleviating a psychotic episode.
- A use or compound for use as defined above wherein the psychosis or psychotic episode arises from or is associated with schizophrenia.
- The use of 3, 11b-*cis*-dihydrotetrabenazine Isomer A for the manufacture of a medicament for preventing, alleviating or reducing one or more symptoms of schizophrenia.

The psychotic episodes, psychoses or symptoms prevented, alleviated or reduced in accordance with the invention may be any one or more symptoms selected from:

- delusions;
- hallucinations;
- visual hallucinations;
- auditory hallucinations;
- hallucinations involving tactile sensations, tastes or smells;
- confusion;
- emotional, behavioral, or intellectual disturbances;
- withdrawal from reality;
- illogical and/or disorganized patterns of thinking;
- paranoid or delusional beliefs;
- paranoia
- grandiose delusions;
- persecutory or self-blaming delusions; and
- personality changes.

The psychotic episodes, psychoses or symptoms prevented, alleviated or reduced in accordance with the invention may be any one or more selected from those arising from or associated with:

- psychosis caused by or associated with schizophrenia;
- psychosis caused by or associated with bipolar disorder (manic depression);
- psychosis caused by or associated with severe clinical depression;
- psychosis induced by disorders and conditions such as:

    o electrolyte disorder;

- o urinary tract infections in the elderly;
- o pain syndromes;
- o drug toxicity;
- o drug withdrawal; and
- o infections of or injuries to the brain;

- psychosis caused by chronic psychological stress (brief reactive psychosis);
- psychosis triggered or exacerbated by severe mental stress; and
- psychosis triggered by or arising from or following illnesses and conditions such as AIDS, leprosy, malaria and mumps.

In one embodiment, the symptoms or psychoses arise from or are associated with schizophrenia and may be any one or more symptoms selected from:

- delusions;
- hallucinations;
- confusion;
- emotional, behavioral, or intellectual disturbances;
- withdrawal from reality; and
- illogical patterns of thinking.

The cis-dihydrotetrabenazine Isomer A used in the invention may be in substantially pure form, for example at an isomeric purity of greater than 90%, typically greater than 95% and more preferably greater than 98%.

The term "isomeric purity" in the present context refers to the amount of 3,11b-*cis*-dihydrotetrabenazine Isomer A present relative to the total amount or concentration of dihydrotetrabenazine of all isomeric forms. For example, if 90% of the total dihydrotetrabenazine present in the composition is 3,11b-*cis*-dihydrotetrabenazine Isomer A, then the isomeric purity is 90%.

The 11b-*cis*-dihydrotetrabenazine Isomer A used in the invention may be in the form of a composition which is substantially free of 3,11b-*trans*- dihydrotetrabenazine, preferably containing less than 5% of 3,11b-*trans*-dihydrotetrabenazine, more preferably less than 3% of 3,11b-*trans*- dihydrotetrabenazine, and most preferably less than 1% of 3,11b-*trans*- dihydrotetrabenazine.

Isomer A can be characterised by its spectroscopic, optical and chromatographic properties, as described in the Examples below, and also by its absolute stereochemical configurations as determined by X-ray crystallography.

Isomer A may be presented in a substantially enantiomerically pure form or as a mixture with other 3,11b-*cis*-dihydrotetrabenazine enantiomers as described herein.

The terms "enantiomeric purity" and "enantiomerically pure" in the present context refer to the amount of 3,11 b-*cis*-dihydrotetrabenazine Isomer A present relative to the total amount or concentration of dihydrotetrabenazine of all enantiomeric and isomeric forms. For example, if 90% of the total dihydrotetrabenazine present in the composition is in the form of a single enantiomer, then the enantiomeric purity is 90%.

By way of example, in each aspect and embodiment of the invention, Isomer A may be present in an enantiomeric purity of at least 55% (e.g. at least 60%, 65%, 70%, 75%, 80%, 85%, 90%, 95%, 97%, 98%, 99%, 99.5% or 100%).

Isomer A may also be presented in the form of mixtures with one or more of Isomers B, C and D. Such mixtures may be racemic mixtures or non-racemic mixtures. Examples of racemic mixtures include the racemic mixture of Isomer A and Isomer B.

## Pharmaceutically Acceptable Salts

Unless the context requires otherwise, a reference in this application to Isomer A includes within its scope not only the free base of the dihydrotetrabenazine but also its salts, and in particular acid addition salts.

Particular acids from which the acid addition salts are formed include acids having a pKa value of less than 3.5 and more usually less than 3. For example, the acid addition salts can be formed from an acid having a pKa in the range from +3.5 to -3.5.

Preferred acid addition salts include those formed with sulphonic acids such as methanesulphonic acid, ethanesulphonic acid, benzene sulphonic acid, toluene sulphonic acid, camphor sulphonic acid and naphthalene sulphonic acid.

One particular acid from which acid addition salts may be formed is methanesulphonic acid.

Acid addition salts can be prepared by the methods described herein or conventional chemical methods such as the methods described in Pharmaceutical Salts: Properties, Selection, and Use, P. Heinrich Stahl (Editor), Camille

G. Wermuth (Editor), ISBN: 3-90639-026-8, Hardcover, 388 pages, August 2002. Generally, such salts can be prepared by reacting the free base form of the compound with the appropriate base or acid in water or in an organic solvent, or in a mixture of the two; generally, nonaqueous media such as ether, ethyl acetate, ethanol, isopropanol, or acetonitrile are used.

The salts are typically pharmaceutically acceptable salts. However, salts that are not pharmaceutically acceptable may also be prepared as intermediate forms which may then be converted into pharmaceutically acceptable salts. Such non-pharmaceutically acceptable salt forms also form part of the invention.

## Methods for the Preparation of Dihydrotetrabenazine Isomers

In this section, methods for preparing all four 3,11 b-cis-dihydrotetrabenazine isomers are described although the invention relates only to the therapeutic uses of the compound of formula (Ib) (Isomer A).

The dihydrotetrabenazines can be prepared by a process comprising the reaction of a compound of the formula (II):

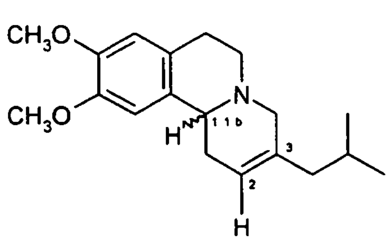

(II)

with a reagent or reagents suitable for hydrating the 2,3-double bond in the compound of formula (II) and thereafter where required separating and isolating a desired dihydrotetrabenazine isomer form.

The hydration of the 2,3-double bond can be carried out by hydroboration using a borane reagent such as diborane or a borane-ether (e.g. borane-tetrahydrofuran (THF)) to give an intermediate alkyl borane adduct followed by oxidation of the alkyl borane adduct and hydrolysis in the presence of a base. The hydroboration is typically carried out in a dry polar non-protic solvent such as an ether (e.g. THF), usually at a non-elevated temperature, for example room temperature. The borane-alkene adduct is typically oxidised with an oxidising agent such as hydrogen peroxide in the presence of a base providing a source of hydroxide ions, such as ammonium hydroxide or an alkali metal hydroxide, e.g. potassium hydroxide or sodium hydroxide. The hydroboration-oxidation-hydrolysis sequence of reactions of Process A typically provides dihydrotetrabenazine isomers in which the hydrogen atoms at the 2- and 3-positions have a *trans* relative orientation.

Compounds of the formula (II) can be prepared by reduction of tetrabenazine to give a dihydrotetrabenazine followed by dehydration of the dihydrotetrabenazine. Reduction of the tetrabenazine can be accomplished using an aluminium hydride reagent such as lithium aluminium hydride, or a borohydride reagent such as sodium borohydride, potassium borohydride or a borohydride derivative, for example an alkyl borohydride such as lithium tri-sec-butyl borohydride. Alternatively, the reduction step can be effected using catalytic hydrogenation, for example over a Raney nickel or platinum oxide catalyst. Suitable conditions for performing the reduction step are described in more detail below or can be found in US 2,843,591 (Hoffmann- La Roche) and Brossi et al., Helv. Chim. Acta., vol. XLI, No. 193, pp1793-1806 (1958).

Because the tetrabenazine used as the starting material for the reduction reaction is typically a mixture of the RR and SS isomers (i.e. *trans*-tetrabenazine), the dihydrotetrabenazine formed by the reduction step will have the same *trans* configuration about the 3- and 11b positions and will take the form of one or more of the known dihydrotetrabenazine isomers shown in Figure 3 above. Thus Process A may involve taking the known isomers of dihydrotetrabenazine, dehydrating them to form the alkene (II) and then "rehydrating" the alkene (II) using conditions that give the required novel *cis* dihydrotetrabenazine isomers of the invention.

Dehydration of the dihydrotetrabenazine to the alkene (II) can be carried out using a variety of standard conditions for dehydrating alcohols to form alkenes, see for example J. March (*idem*) pages 389-390 and references therein. Examples of such conditions include the use of phosphorus-based dehydrating agents such as phosphorus halides or phosphorus oxyhalides, e.g. $POCl_3$ and $PCl_5$. As an alternative to direct dehydration, the hydroxyl group of the dihydrotetrabenazine can be converted to a leaving group L such as halogen (e.g. chlorine or bromine) and then subjected to conditions (e.g. the presence of a base) for eliminating H-L. Conversion of the hydroxyl group to a halide can be achieved using methods well known to the skilled chemist, for example by reaction with carbon tetrachloride or carbon tetrabromide in the presence of a trialkyl or triaryl phosphine such as triphenyl phosphine or tributyl phosphine.

The tetrabenazine used as the starting material for the reduction to give the dihydrotetrabenazine can be obtained commercially or can be synthesised by the method described in US 2,830,993 (Hoffmann-La Roche).

Another process (Process B) for preparing a 3,11 b-cis-dihydrotetrabenazine comprises subjecting a compound of the formula (III):

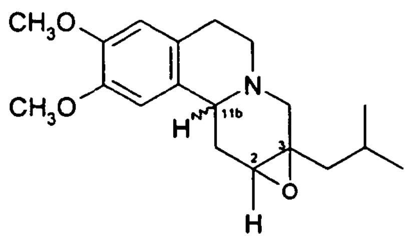

(III)

to conditions for ring-opening the 2,3-epoxide group in the compound of the formula (III), and thereafter where required separating and isolating a desired dihydrotetrabenazine isomer form.

The ring-opening can be effected in accordance with known methods for epoxide ring openings. However, a currently preferred method of ring-opening the epoxide is reductive ring opening which can be achieved using a reducing agent such as borane-THF. Reaction with borane-THF can be carried out in a polar non-protic solvent such as ether (e.g. tetrahydrofuran) usually at ambient temperature, the borane complex thus formed being subsequently hydrolysed by heating in the presence of water and a base at the reflux temperature of the solvent. Process B typically gives rise to dihydrotetrabenazine isomers in which the hydrogen atoms at the 2- and 3-positions have a *cis* relative orientation.

The epoxide compounds of the formula (III) can be prepared by epoxidation of an alkene of the formula (II) above. The epoxidation reaction can be carried out using conditions and reagents well known to the skilled chemist, see for example J. March (*idem*), pages 826-829 and references therein. Typically, a per-acid such as *meta*-chloroperbenzoic acid (MCPBA), or a mixture of a per-acid and a further oxidising agent such as perchloric acid, may be used to bring about epoxidation.

When the starting materials for processes A and B above are mixtures of enantiomers, then the products of the processes will typically be pairs of enantiomers, for example racemic mixtures, possibly together with diastereoisomeric impurities. Unwanted diastereoisomers can be removed by techniques such as chromatography (e.g. HPLC) and the individual enantiomers can be separated by a variety of methods known to the skilled chemist. For example, they can be separated by means of:

(i) chiral chromatography (chromatography on a chiral support); or

(ii) forming a salt with an optically pure chiral acid, separating the salts of the two diastereoisomers by fractional crystallisation and then releasing the dihydrotetrabenazine from the salt; or

(iii) forming a derivative (such as an ester) with an optically pure chiral derivatising agent (e.g. esterifying agent), separating the resulting epimers (e.g. by chromatography) and then converting the derivative to the dihydrotetrabenazine.

One method of separating pairs of enantiomers obtained from each of Processes A and B, and which has been found to be particularly effective, is to esterify the hydroxyl group of the dihydrotetrabenazine with an optically active form of Mosher's acid, such as the *R* (+) isomer shown below, or an active form thereof:

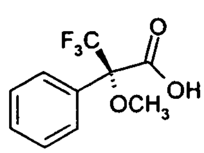

The resulting esters of the two enantiomers of the dihydrobenazine can then be separated by chromatography (e.g. HPLC) and the separated esters hydrolysed to give the individual dihydrobenazine isomers using a base such as an alkali metal hydroxide (e.g. NaOH) in a polar solvent such as methanol.

As an alternative to using mixtures of enantiomers as the starting materials in processes A and B and then

carrying out separation of enantiomers subsequently, processes A and B can each be carried out on single enantiomer starting materials leading to products in which a single enantiomer predominates. Single enantiomers of the alkene (II) can be prepared by subjecting RR/SS tetrabenazine to a stereoselective reduction using lithium tri-sec-butyl borohydride to give a mixture of SRR and RSS enantiomers of dihydrotetrabenazine, separating the enantiomers (e.g. by fractional crystallisation) and then dehydrating a separated single enantiomer of dihydrotetrabenazine to give predominantly or exclusively a single enantiomer of the compound of formula (II).

Processes A and B are illustrated in more detail below in Schemes 1 and 2 respectively.

Scheme 1

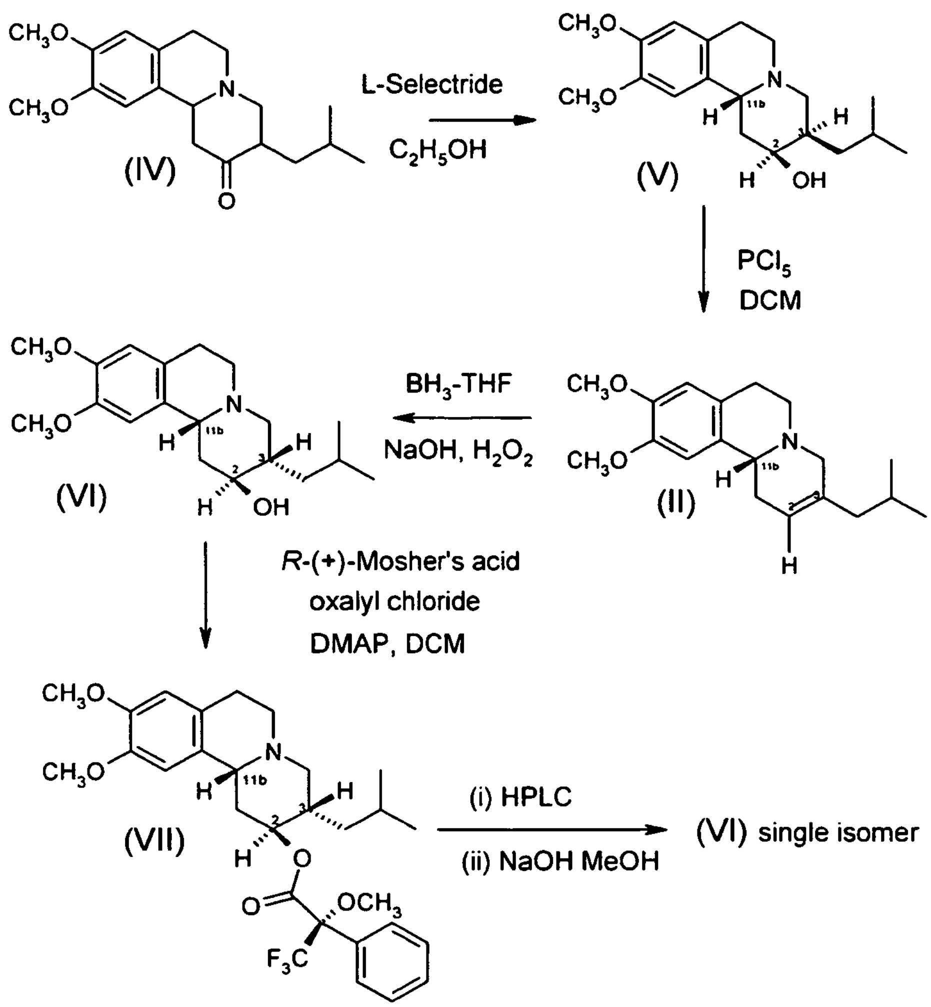


Scheme 1 illustrates the preparation of individual dihydrotetrabenazine isomers having the 2S,3S, 11b*R* and 2*R*,3*R*, 11b*S* configurations in which the hydrogen atoms attached to the 2- and 3-positions are arranged in a *trans* relative orientation. This reaction scheme includes Process A defined above.

The starting point for the sequence of reactions in Scheme 1 is commercially available tetrabenazine (IV) which is a racemic mixture of the RR and SS optical isomers of tetrabenazine. In each of the RR and SS isomers, the hydrogen atoms at the 3- and 11b-positions are arranged in a *trans* relative orientation. As an alternative to using the commercially available compound, tetrabenazine can be synthesised according to the procedure described in US patent number 2,830,993 (see in particular example 11).

The racemic mixture of RR and SS tetrabenazine is reduced using the borohydride reducing agent lithium tri-sec-butyl borohydride ("L-Selectride") to give a mixture of the known 2*S*,3*R*, 11b*R* and 2*R*,3S, 11b*S* isomers (V) of dihydrotetrabenazine, of which only the 2*S*,3*R*,11b*R* isomer is shown for simplicity. By using the more sterically demanding L-Selectride as the borohydride reducing agent rather than sodium borohydride, formation of the RRR and

SSS isomers of dihydrotetrabenazine is minimised or suppressed.

The dihydrotetrabenazine isomers (V) are reacted with a dehydrating agent such as phosphorus pentachloride in a non-protic solvent such as a chlorinated hydrocarbon (for example chloroform or dichloromethane, preferably dichloromethane) to form the unsaturated compound (II) as a pair of enantiomers, only the *R*-enantiomer of which is shown in the Scheme. The dehydration reaction is typically carried out at a temperature lower than room temperature, for example at around 0-5˚C.

The unsaturated compound (II) is then subjected to a stereoselective re-hydration to generate the dihydrotetrabenazine (VI) and its mirror image or antipode (not shown) in which the hydrogen atoms at the 3- and 11b-positions are arranged in a *cis* relative orientation and the hydrogen atoms at the 2- and 3-positions are arranged in a *trans* relative orientation. The stereoselective rehydration is accomplished by a hydroboration procedure using borane-THF in tetrahydrofuran (THF) to form an intermediate borane complex (not shown) which is then oxidised with hydrogen peroxide in the presence of a base such as sodium hydroxide.

An initial purification step may then be carried out (e.g. by HPLC) to give the product (V) of the rehydration reaction sequence as a mixture of the 2*S*,3*S*, 11b*R* and 2*R*,3*R*,11b*S* isomers of which only the 2*S*,3*S*, 11b*R* isomer is shown in the Scheme. In order to separate the isomers, the mixture is treated with R (+) Mosher's acid, in the presence of oxalyl chloride and dimethylaminopyridine (DMAP) in dichloromethane to give a pair of diastereoisomeric esters (VII) (of which only one diastereoisomer is shown) which can then be separated using HPLC. The individual esters can then be hydrolysed using an alkali metal hydroxide such as sodium hydroxide to give a single isomer (VI).

In a variation of the sequence of steps shown in Scheme 1, following the reduction of RR/SS tetrabenazine, the resulting mixture of enantiomers of the dihydrotetrabenazine (V) can be separated to give the individual enantiomers. Separation can be carried out by forming a salt with a chiral acid such as (+) or (-) camphorsulphonic acid, separating the resulting diastereoisomers by fractional crystallisation to give a salt of a single enantiomer and then releasing the free base from the salt.

The separated dihydrotetrabenazine enantiomer can be dehydrated to give a single enantiomer of the alkene (II). Subsequent rehydration of the alkene (II) will then give predominantly or exclusively a single enantiomer of the cis-dihydrotetrabenazine (VI). An advantage of this variation is that it does not involve the formation of Mosher's acid esters and therefore avoids the chromatographic separation typically used to separate Mosher's acid esters.

Scheme 2 illustrates the preparation of individual dihydrotetrabenazine isomers having the 2*R*,3*S*, 11b*R* and 2*S*,3*R*,11b*S* configurations in which the hydrogen atoms attached to the 2- and 3-positions are arranged in a *cis* relative orientation. This reaction scheme includes Process B defined above.

## Scheme 2

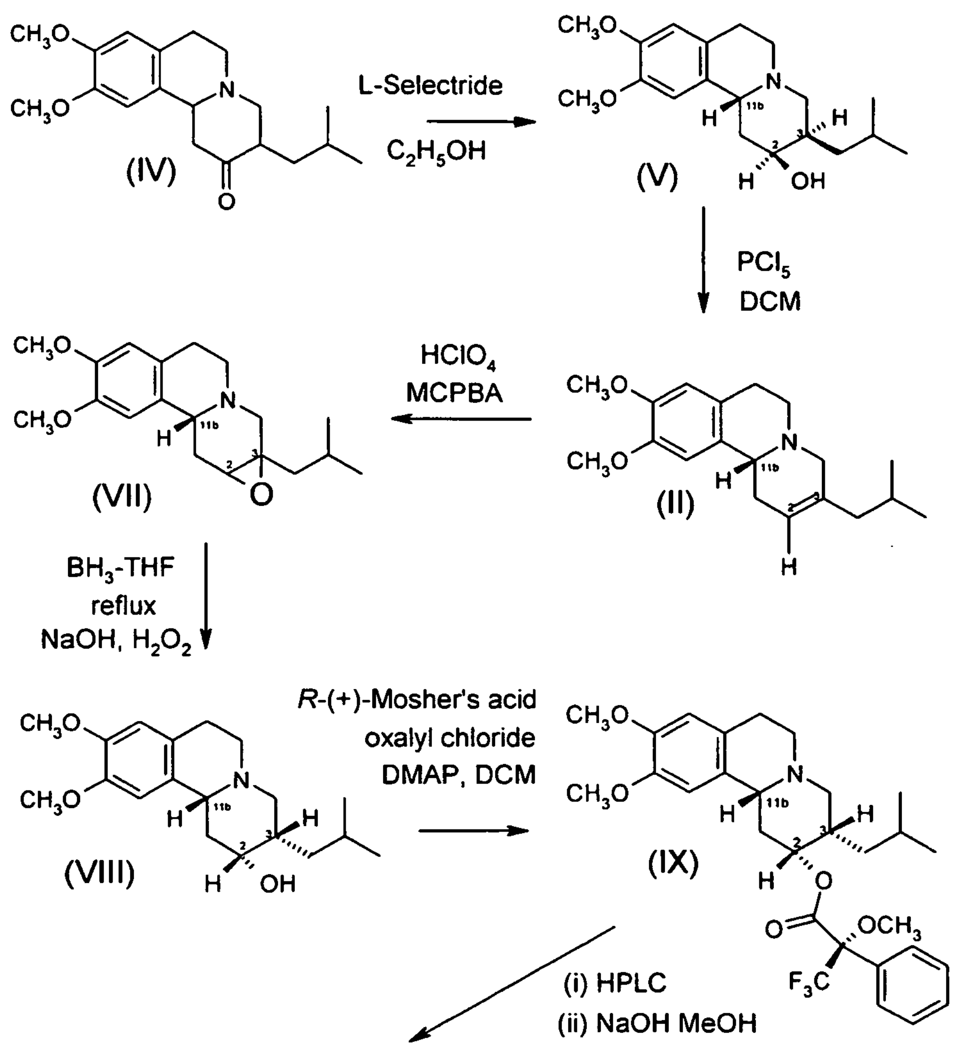


(VIII) single isomer

In Scheme 2, the unsaturated compound (II) is produced by reducing tetrabenazine to give the 2S,3R,11bR and 2R,3S,11bS isomers (V) of dihydrotetrabenazine and dehydrating with $PCl_5$ in the manner described above in Scheme 1. However, instead of subjecting the compound (II) to hydroboration, the 2,3-double bond is converted to an epoxide by reaction with *meta*-chloroperbenzoic acid (MCPBA) and perchloric acid. The epoxidation reaction is conveniently carried out in an alcohol solvent such as methanol, typically at around room temperature.

The epoxide (VII) is then subjected to a reductive ring opening using borane-THF as an electrophilic reducing agent to give an intermediate borane complex (not shown) which is then oxidised and cleaved with hydrogen peroxide in the presence of an alkali such as sodium hydroxide to give a dihydrotetrabenazine (VIII) as a mixture of the 2*R*,3*S*, 11b*R* and 2*S*,3*R*, 11b*S* isomers, of which only the 2*R*,3*S*, 11b*R* is shown for simplicity. Treatment of the mixture of isomers (VIII) with R (+) Mosher's acid in the presence of oxalyl chloride and dimethylaminopyridine (DMAP) in dichloromethane gives a pair of epimeric esters (IX) (of which only one epimer is shown) which can then by separated by chromatography and hydrolysed with sodium hydroxide in methanol in the manner described above in relation to Scheme 1.

### Pharmaceutical Formulations

The cis-dihydrotetrabenazine compound of the invention is typically administered in the form of a pharmaceutical composition.

The pharmaceutical compositions can be in any form suitable for oral, parenteral, topical, intranasal, intrabronchial, ophthalmic, otic, rectal, intra-vaginal, or transdermal administration. Where the compositions are intended for parenteral administration, they can be formulated for intravenous, intramuscular, intraperitoneal, subcutaneous administration or for direct delivery into a target organ or tissue by injection, infusion or other means of delivery.

Pharmaceutical dosage forms suitable for oral administration include tablets, capsules, caplets, pills, lozenges, syrups, solutions, sprays, powders, granules, elixirs and suspensions, sublingual tablets, sprays, wafers or patches and buccal patches.

Pharmaceutical compositions containing the dihydrotetrabenazine compound of the invention can be formulated in accordance with known techniques, see for example, Remington's Pharmaceutical Sciences, Mack Publishing Company, Easton, PA, USA.

Thus, tablet compositions can contain a unit dosage of active compound together with an inert diluent or carrier such as a sugar or sugar alcohol, e.g.; lactose, sucrose, sorbitol or mannitol; and/or a non-sugar derived diluent such as sodium carbonate, calcium phosphate, talc, calcium carbonate, or a cellulose or derivative thereof such as methyl cellulose, ethyl cellulose, hydroxypropyl methyl cellulose, and starches such as corn starch. Tablets may also contain such standard ingredients as binding and granulating agents such as polyvinylpyrrolidone, disintegrants (e.g. swellable crosslinked polymers such as crosslinked carboxymethylcellulose), lubricating agents (e.g. stearates), preservatives (e.g. parabens), antioxidants (e.g. BHT), buffering agents (for example phosphate or citrate buffers), and effervescent agents such as citrate/bicarbonate mixtures. Such excipients are well known and do not need to be discussed in detail here.

Capsule formulations may be of the hard gelatin or soft gelatin variety and can contain the active component in solid, semi-solid, or liquid form. Gelatin capsules can be formed from animal gelatin or synthetic or plant derived equivalents thereof.

The solid dosage forms (e.g.; tablets, capsules etc.) can be coated or un-coated, but typically have a coating, for example a protective film coating (e.g. a wax or varnish) or a release controlling coating. The coating (e.g. a Eudragit ™ type polymer) can be designed to release the active component at a desired location within the gastro-intestinal tract. Thus, the coating can be selected so as to degrade under certain pH conditions within the gastrointestinal tract, thereby selectively release the compound in the stomach or in the ileum or duodenum.

Instead of, or in addition to, a coating, the drug can be presented in a solid matrix comprising a release controlling agent, for example a release delaying agent which may be adapted to selectively release the compound under conditions of varying acidity or alkalinity in the gastrointestinal tract. Alternatively, the matrix material or release retarding coating can take the form of an erodible polymer (e.g. a maleic anhydride polymer) which is substantially continuously eroded as the dosage form passes through the gastrointestinal tract.

Compositions for topical use include ointments, creams, sprays, patches, gels, liquid drops and inserts (for example intraocular inserts). Such compositions can be formulated in accordance with known methods.

Compositions for parenteral administration are typically presented as sterile aqueous or oily solutions or fine suspensions, or may be provided in finely divided sterile powder form for making up extemporaneously with sterile water for injection.

Examples of formulations for rectal or intra-vaginal administration include pessaries and suppositories which may be, for example, formed from a shaped mouldable or waxy material containing the active compound.

Compositions for administration by inhalation may take the form of inhalable powder compositions or liquid or powder sprays, and can be administrated in standard form using powder inhaler devices or aerosol dispensing devices. Such devices are well known. For administration by inhalation, the powdered formulations typically comprise the active compound together with an inert solid powdered diluent such as lactose.

The compound of the invention will generally be presented in unit dosage form and, as such, will typically contain sufficient compound to provide a desired level of biological activity. For example, a formulation intended for oral administration may contain from 2 milligrams to 200 milligrams of active ingredient, more usually from 10 milligrams to 100 milligrams, for example, 12.5 milligrams, 25 milligrams and 50 milligrams.

## Methods of Treatment

The active compound will be administered to a patient in need thereof (for example a human or animal patient) in an amount sufficient to achieve the desired therapeutic effect.

The patient in need of such administration is a patient suffering from or exhibiting, or at risk of suffering from or exhibiting, one or more psychoses, for example a psychosis characteristic of schizophrenia.

The desired effect can be the prevention, alleviation or reduction of the severity of the psychosis or one or more symptoms thereof. Such symptoms are well known to the skilled person (e.g. a skilled physician) who will be able to judge through clinical evaluation and testing in a conventional manner whether or not the administration of a compound of the invention has resulted in a change in the symptoms exhibited by the patient.

The compound will typically be administered in amounts that are therapeutically or prophylactically useful and which generally are non-toxic. However, in certain situations, the benefits of administering the dihydrotetrabenazine compound of the invention may outweigh the disadvantages of any toxic effects or side effects, in which case it may be considered desirable to administer compounds in amounts that are associated with a degree of toxicity.
A typical daily dose of the compound can be up to 1000 mg per day, for example in the range from 0.01 milligrams to 10 milligrams per kilogram of body weight, more usually from 0.025 milligrams to 5 milligrams per kilogram of body weight, for example up to 3 milligrams per kilogram of bodyweight, and more typically 0.15 milligrams to 5 milligrams per kilogram of bodyweight although higher or lower doses may be administered where required.
Ultimately, however, the quantity of compound administered will be commensurate with the nature of the disease or physiological condition being treated and the therapeutic benefits and the presence or absence of side effects produced by a given dosage regimen, and will be at the discretion of the physician.

## EXAMPLES

The following non-limiting examples illustrate the synthesis and properties of the 3,11 b-cis-dihydrotetrabenazine isomers. The examples describe all four isomers of 3,11b-*cis*-dihydrotetrabenazine although the invention is limited to the therapeutic uses of Isomer A (the compound of formula (Ib)). The examples relating to the other isomers are retained as comparative examples.

EXAMPLE 1

Preparation of 2*S*,3*S*,11b*R* and 2*R*,3*R*,11b*S* Isomers of Dihydrotetrabenazine

1A. Reduction of RR/SS Tetrabenazine

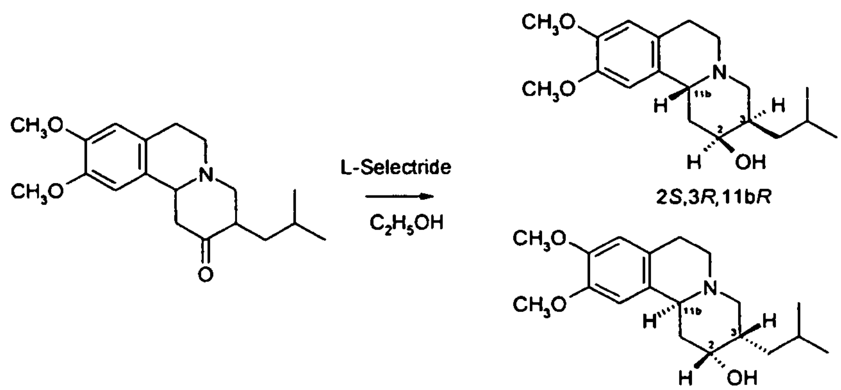


2*S*,3*R*,11b*R*

2*R*,3*S*,11b*S*

1M L-Selectride® in tetrahydrofuran (135 ml, 135 mmol, 2.87 eq.) was added slowly over 30 minutes to a stirred solution of tetrabenazine RR/SS racemate (15 g, 47 mmol) in ethanol (75 ml) and tetrahydrofuran (75 ml) at 0 ˚C. After addition was complete the mixture was stirred at 0 ˚C for 30 minutes and then allowed to warm to room temperature.
The mixture was poured onto crushed ice (300 g) and water (100 ml) added. The solution was extracted with diethyl ether (2 x 200 ml) and the combined ethereal extracts washed with water (100 ml) and partly dried over anhydrous potassium carbonate. Drying was completed using anhydrous magnesium sulphate and, after filtration, the solvent was removed at reduced pressure (shielded from the light, bath temperature <20 ˚C) to afford a pale yellow solid.
The solid was slurried with petroleum ether (30-40 ˚C) and filtered to afford a white powdery solid (12 g, 80%).

1B. Dehydration of reduced Tetrabenazine

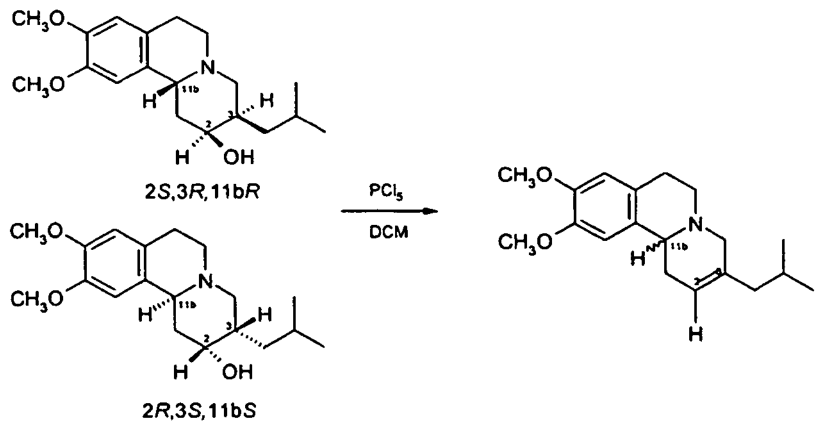


2S,3R,11bR

2R,3S,11bS

Phosphorous pentachloride (32.8 g, 157.5 mmol, 2.5 eq) was added in portions over 30 minutes to a stirred solution of the reduced tetrabenazine product from Example 1A (20 g, 62.7 mmol) in dichloromethane (200 ml) at 0 °C. After the addition was complete, the reaction mixture was stirred at 0 °C for a further 30 minutes and the solution poured slowly into 2M aqueous sodium carbonate solution containing crushed ice (0 °C). Once the initial acid gas evolution had ceased the mixture was basified (ca. pH 12) using solid sodium carbonate.

The alkaline solution was extracted using ethyl acetate (800 ml) and the combined organic extracts dried over anhydrous magnesium sulphate. After filtration the solvent was removed at reduced pressure to afford a brown oil, which was purified by column chromatography (silica, ethyl acetate) to afford the semi-pure alkene as a yellow solid (10.87 g, 58%).

1C. Hydration of the Crude Alkene from Example 1B

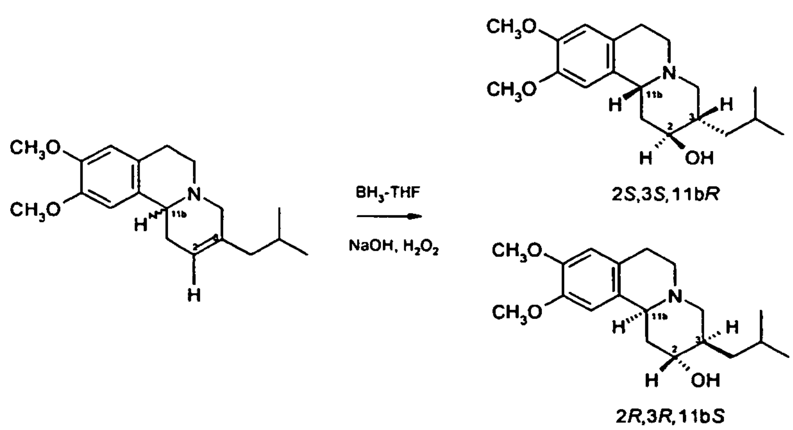


2S,3S,11bR

2R,3R,11bS

A solution of the crude alkene (10.87 g, 36.11 mmol) from Example 1B in dry THF (52 ml) at room temperature was treated with 1M borane-THF (155.6 ml, 155.6 mmol, 4.30 eq) added in a dropwise manner. The reaction was stirred for 2 hours, water (20 ml) was added and the solution basified to pH 12 with 30% aqueous sodium hydroxide solution.

Aqueous 30% hydrogen peroxide solution (30 ml) was added to the stirred alkaline reaction mixture and the solution was heated to reflux for 1 hour before being allowed to cool. Water (100 ml) was added and the mixture extracted with ethyl acetate (3 x 250 ml). The organic extracts were combined and dried over anhydrous magnesium sulphate and after filtration the solvent was removed at reduced pressure to afford a yellow oil (9 g).

The oil was purified using preparative HPLC (Column: Lichrospher Si60, 5 μm, 250 x 21.20 mm, mobile phase: hexane : ethanol : dichloromethane (85:15:5); UV 254 nm, flow: 10 ml min$^{-1}$) at 350 mg per injection followed by concentration of the fractions of interest under vacuum. The product oil was then dissolved in ether and concentrated once more under vacuum to give the dihydrotetrabenazine racemate shown above as a yellow foam (5.76 g, 50%).

1D. Preparation of Mosher's ester derivatives

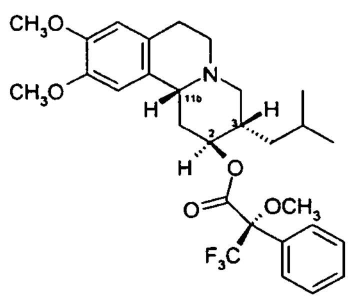

and

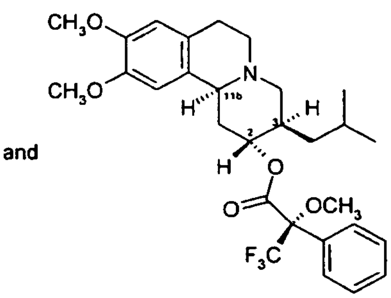

R-(+)-α-methoxy-α-trifluoromethylphenyl acetic acid (5 g, 21.35 mmol), oxalyl chloride (2.02 ml) and DMF (0.16 ml) were added to anhydrous dichloromethane (50 ml) and the solution was stirred at room temperature for 45 minutes. The solution was concentrated under reduced pressure and the residue was taken up in anhydrous dichloromethane (50 ml) once more. The resulting solution was cooled using an ice-water bath and dimethylaminopyridine (3.83 g, 31.34 mmol) was added followed by a pre-dried solution (over 4Å sieves) in anhydrous dichloromethane of the solid product of Example 1C (5 g, 15.6 mmol). After stirring at room temperature for 45 minutes, water (234 ml) was added and the mixture extracted with ether (2 x 200 ml). The ether extract was dried over anhydrous magnesium sulphate, passed through a pad of silica and the product eluted using ether.

The collected ether eluate was concentrated under reduced pressure to afford an oil which was purified using column chromatography (silica, hexane : ether (10:1)). Evaporation of the collected column fractions of interest and removal of the solvent at reduced pressure gave a solid which was further purified using column chromatography (silica, hexane : ethyl acetate (1:1)) to give three main components which were partially resolved into Mosher's ester peaks 1 and 2.

Preparative HPLC of the three components (Column: 2 x Lichrospher Si60, 5 μm, 250 x 21.20 mm, mobile phase: hexane : isopropanol (97:3), UV 254 nm; flow: 10 ml $min^{-1}$) at 300 mg loading followed by concentration of the fractions of interest under vacuum gave the pure Mosher's ester derivatives

Peak 1 (3.89 g, 46.5%)
Peak 2 (2.78 g, 33%)

The fractions corresponding to the two peaks were subjected to hydrolysis to liberate the individual dihydrotetrabenazine isomers identified and characterised as Isomers A and B. Isomers A and B are each believed to have one of the following structures

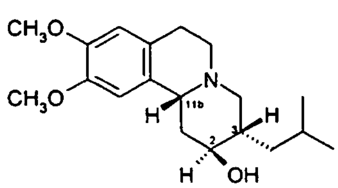

2*S*,3*S*,11b*R*

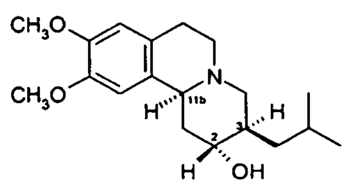

2*R*,3*R*,11b*S*

More specifically, Isomer B is believed to have the 2S, 3S, 11b*R* absolute configuration on the basis of the X-ray crystallography experiments described in Example 4 below.

1E. Hydrolysis of Peak 1 to give Isomer A

Aqueous 20% sodium hydroxide solution (87.5 ml) was added to a solution of Mosher's ester peak 1 (3.89 g, 7.27 mmol) in methanol (260 ml) and the mixture stirred and heated to reflux for 150 minutes. After cooling to room

temperature water (200 ml) was added and the solution extracted with ether (600 ml), dried over anhydrous magnesium sulphate and after filtration, concentrated under reduced pressure.

The residue was dissolved using ethyl acetate (200 ml), the solution washed with water (2 x 50 ml), the organic phase dried over anhydrous magnesium sulphate and after filtration, concentrated under reduced pressure to give a yellow foam. This material was purified by column chromatography (silica, gradient elution of ethyl acetate : hexane (1: 1) to ethyl acetate). The fractions of interest were combined and the solvent removed at reduced pressure. The residue was taken up in ether and the solvent removed at reduced pressure once more to give Isomer A as an off-white foam (1.1 g, 47%).

Isomer A, which is believed to have the 2*R*,3*R*,11b*S* configuration (the absolute stereochemistry was not determined), was characterized by $^{1}$H-NMR, $^{13}$C-NMR, IR, mass spectrometry, chiral HPLC and ORD. The IR, NMR and MS data for isomer A are set out in Table 1 and the Chiral HPLC and ORD data are set out in Table 3.

1F. Hydrolysis of Peak 2 to give Isomer B

Aqueous 20% sodium hydroxide solution (62.5 ml) was added to a solution of Mosher's ester peak 2 (2.78 g, 5.19 mmol) in methanol (185 ml) and the mixture stirred and heated to reflux for 150 minutes. After cooling to room temperature water (142 ml) was added and the solution extracted with ether (440 ml), dried over anhydrous magnesium sulphate and after filtration, concentrated under reduced pressure.

The residue was dissolved using ethyl acetate (200 ml), the solution washed with water (2 x 50 ml), the organic phase dried over anhydrous magnesium sulphate and after filtration, concentrated under reduced pressure. Petroleum ether (30-40 °C) was added to the residue and the solution concentrated under vacuum once more to give Isomer B as a white foam (1.34 g, 81%).

Isomer B, which is believed to have the 2*S*,3*S*,11b*R* configuration, was characterized by $^{1}$H-NMR, $^{13}$C-NMR, IR, mass spectrometry, chiral HPLC, ORD and X-ray crystallography. The IR, NMR and MS data for Isomer B are set out in Table 1 and the Chiral HPLC and ORD data are set out in Table 3. The X-ray crystallography data are set out in Example 4.

EXAMPLE 2

Preparation of 2*R*,3*S*, 11b*R* and 2*S*,3*R*,11b*S* Isomers of Dihydrotetrabenazine

2A. Preparation of 2,3-Dehydrotetrabenazine

A solution containing a racemic mixture (15 g, 47 mmol) of *RR* and *SS* tetrabenazine enantiomers in tetrahydrofuran was subjected to reduction with L-Selectride® by the method of Example 1A to give a mixture of the 2*S*,3*R*, 11b*R* and 2*R*,3*S*,11b*S* enantiomers of dihydrotetrabenazine.as a white powdery solid (12 g, 80%). The partially purified dihydrotetrabenazine was then dehydrated using $PCl_5$ according to the method of Example 1B to give a semi-pure mixture of 11b*R* and 11 bS isomers of 2,3-dehydrotetrabenazine (the 11b*R* enantiomer of which is shown below) as a yellow solid (12.92 g, 68%).

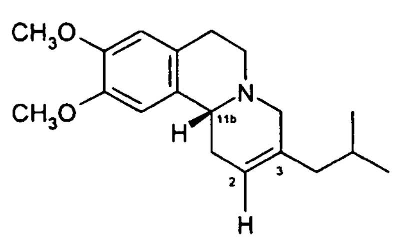

2B. Epoxidation of the Crude Alkene from Example 2A

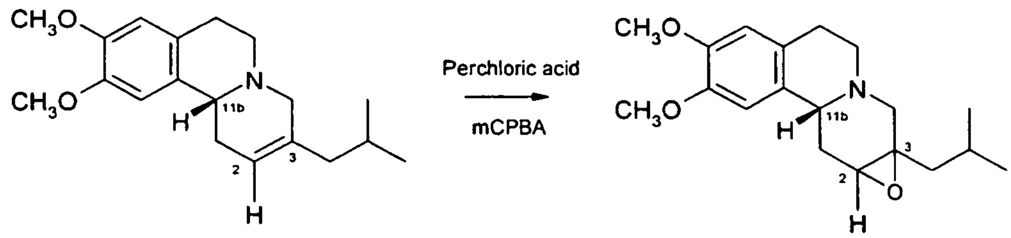


To a stirred solution of the crude alkene from Example 2A (12,92 g, 42.9 mmol) in methanol (215 ml) was added a solution of 70% perchloric acid (3.70 ml, 43 mmol) in methanol (215 ml). 77% 3-Chloroperoxybenzoic acid (15.50 g, 65 mmol) was added to the reaction and the resulting mixture was stirred for 18 hours at room temperature protected from light.

The reaction mixture was poured into saturated aqueous sodium sulphite solution (200 ml) and water (200 ml) added. Chloroform (300 ml) was added to the resulting emulsion and the mixture basified with saturated aqueous sodium bicarbonate (400 ml).

The organic layer was collected and the aqueous phase washed with additional chloroform (2 x 150 ml). The combined chloroform layers were dried over anhydrous magnesium sulphate and after filtration the solvent was removed at reduced pressure to give a brown oil (14.35 g, yield > 100% - probable solvent remains in product). This material was used without further purification.

<u>2C. Reductive Ring Opening of the Epoxide from 2B</u>

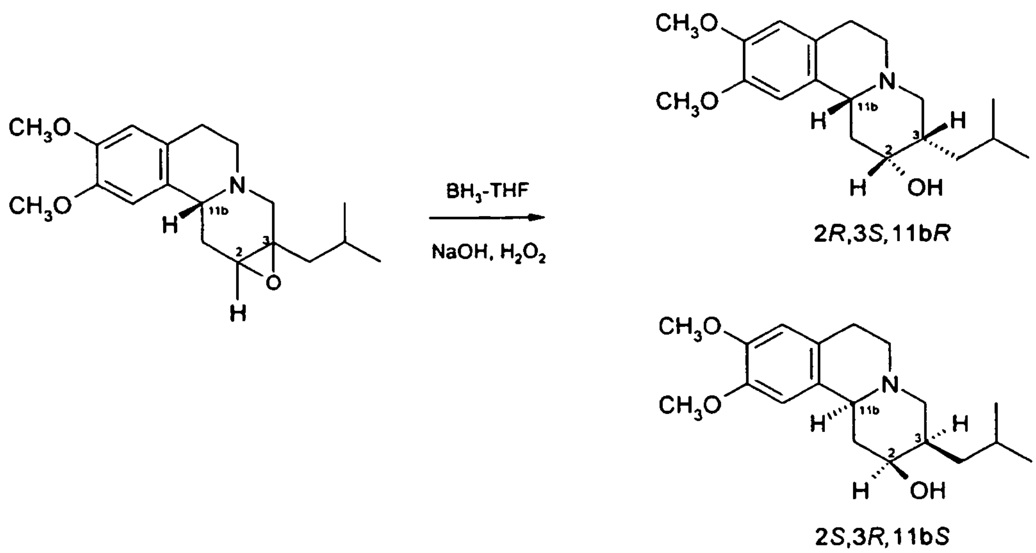


2*R*,3*S*,11b*R*

2*S*,3*R*,11b*S*

A stirred solution of the crude epoxide from Example 2B (14.35 g, 42.9 mmol, assuming 100% yield) in dry THF (80 ml) was treated slowly with 1M borane/THF (184.6 ml, 184.6 mmol) over 15 minutes. The reaction was stirred for two hours, water (65 ml) was added and the solution heated with stirring to reflux for 30 minutes.

After cooling, 30% sodium hydroxide solution (97 ml) was added to the reaction mixture followed by 30% hydrogen peroxide solution (48.6 ml) and the reaction was stirred and heated to reflux for an additional 1 hour.

The cooled reaction mixture was extracted with ethyl acetate (500 ml) dried over anhydrous magnesium sulphate and after filtration the solvent was removed at reduced pressure to give an oil. Hexane (230 ml) was added to the oil and the solution re-concentrated under reduced pressure.

The oily residue was purified by column chromatography (silica, ethyl acetate). The fractions of interest were combined and the solvent removed under reduced pressure. The residue was purified once more using column chromatography (silica, gradient, hexane to ether). The fractions of interest were combined and the solvents evaporated at reduced pressure to give a pale yellow solid (5.18 g, 38%).

2D. Preparation of Mosher's ester derivatives of the 2*R*,3*S*, 11b*R* and 2*S*,3*R*,11b*S* Isomers of Dihydrotetrabenazine

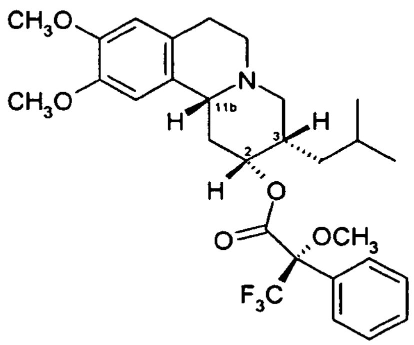

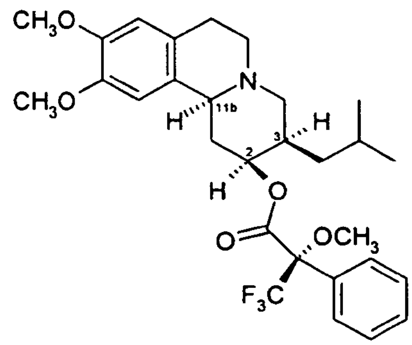

R-(+)-α-methoxy-α-trifluoromethylphenyl acetic acid (4.68 g, 19.98 mmol), oxalyl chloride (1.90 ml) and DMF (0.13 ml) were added to anhydrous dichloromethane (46 ml) and the solution stirred at room temperature for 45 minutes. The solution was concentrated under reduced pressure and the residue was taken up in anhydrous dichloromethane (40 ml) once more. The resulting solution was cooled using an ice-water bath and dimethylaminopyridine (3.65 g, 29.87 mmol) was added followed by a pre-dried solution (over 4Å sieves) in anhydrous dichloromethane (20 ml) of the solid product of Example 2C (4.68 g, 14.6 mmol). After stirring at room temperature for 45 minutes, water (234 ml) was added and the mixture extracted with ether (2 x 200 ml). The ether extract was dried over anhydrous magnesium sulphate, passed through a pad of silica and the product eluted using ether.

The collected ether eluate was concentrated under reduced pressure to afford an oil which was purified using column chromatography (silica, hexane : ether (1:1)).

Evaporation of the collected column fractions of interest and removal of the solvent at reduced pressure gave a pink solid (6.53 g)

Preparative HPLC of the solid (Column: 2 x Lichrospher Si60, 5 μm, 250 x 21.20 mm; mobile phase hexane : isopropanol (97:3); UV 254 nm; flow: 10 ml $min^{-1}$) at 100 mg loading followed by concentration of the fractions of interest under vacuum gave a solid which was slurried with petroleum ether (30-40 °C) and collected by filtration to give the pure Mosher's ester derivatives

Peak 1 (2.37 g, 30%)

Peak 2 (2.42 g, 30%)

The fractions corresponding to the two peaks were subjected to hydrolysis to liberate the individual dihydrotetrabenazine isomers identified and characterised as Isomers C and D. Isomers C and D are each believed to have one of the following structures

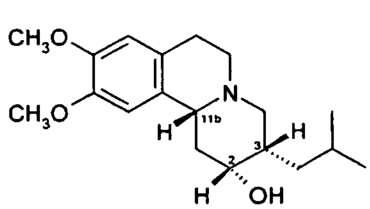

2*R*,3*S*,11b*R*

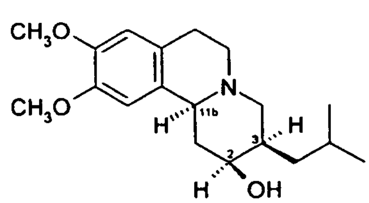

2*S*,3*R*,11b*S*

2F. Hydrolysis of Peak 1 to give Isomer C

20% aqueous sodium hydroxide solution (53 ml) was added to a stirred solution of Mosher's ester peak 1 (2.37 g, 4.43 mmol) in methanol (158 ml) and the mixture stirred at reflux for 150 minutes. After cooling water (88 ml) was added to the reaction mixture and the resulting solution extracted with ether (576 ml). The organic extract was dried over anhydrous magnesium sulphate and after filtration the solvent removed at reduced pressure. Ethyl acetate (200 ml) was added to the residue and the solution washed with water (2 x 50 ml). The organic solution was dried over anhydrous magnesium sulphate and after filtration the solvent removed at reduced pressure.

This residue was treated with petroleum ether (30-40 ˚C) and the resulting suspended solid collected by filtration. The filtrate was concentrated at reduced pressure and the second batch of suspended solid was collected by filtration. Both collected solids were combined and dried under reduced pressure to give Isomer C (1.0g, 70%).

Isomer C, which is believed to have either the 2*R*,3*S*, 11b*R* or *2S,3R,* 11b*S* configuration (the absolute stereochemistry was not determined), was characterized by $^{1}$H-NMR, $^{13}$C-NMR, IR, mass spectrometry, chiral HPLC and ORD. The IR, NMR and MS data for Isomer C are set out in Table 2 and the Chiral HPLC and ORD data are set out in Table 4.

2G. Hydrolysis of Peak 2 to give Isomer D

20% aqueous sodium hydroxide solution (53 ml) was added to a stirred solution of Mosher's ester peak 2 (2.42 g, 4.52 mmol) in methanol (158 ml) and the mixture stirred at reflux for 150 minutes. After cooling water (88 ml) was added to the reaction mixture and the resulting solution extracted with ether (576 ml). The organic extract was dried over anhydrous magnesium sulphate and after filtration the solvent removed at reduced pressure. Ethyl acetate (200 ml) was added to the residue and the solution washed with water (2 x 50 ml). The organic solution was dried over anhydrous magnesium sulphate and after filtration the solvent removed at reduced pressure.

This residue was treated with petroleum ether (30-40 ˚C) and the resulting suspended orange solid collected by filtration. The solid was dissolved in ethyl acetate : hexane (15:85) and purified by column chromatography (silica, gradient ethyl acetate : hexane (15:85) to ethyl acetate). The fractions of interest were combined and the solvent removed at reduced pressure. The residue was slurried with petroleum ether (30-40 ˚C) and the resulting suspension collected by filtration. The collected solid was dried under reduced pressure to give Isomer D as a white solid (0.93 g, 64%).

Isomer D, which is believed to have either the 2*R*,3*S*,11b*R* or 2*S*,3*R*,11b*S* configuration (the absolute stereochemistry was not determined), was characterized by $^{1}$H-NMR, $^{13}$C-NMR, IR, mass spectrometry, chiral HPLC and ORD. The IR, NMR and MS data for Isomer D are set out in Table 2 and the Chiral HPLC and ORD data are set out in Table 4.

In Tables 1 and 2, the infra red spectra were determined using the KBr disc method. The $^{1}$H NMR spectra were carried out on solutions in deuterated chloroform using a Varian Gemini NMR spectrometer (200 MHz.). The $^{13}$C NMR spectra were carried out on solutions in deuterated chloroform using a Varian Gemini NMR spectrometer (50MHz). The mass spectra were obtained using a Micromass Platform II (ES$^{+}$ conditions) spectrometer. In Tables 3 and 4, the Optical Rotatory Dispersion figures were obtained using an Optical Activity PolAAr 2001 instrument in methanol solution at 24˚C. The HPLC retention time measurements were carried out using an HP1050 HPLC chromatograph with UV detection.

Tables 1 and 2 - Spectroscopic Data

| Table 1 | | | | |
|---|---|---|---|---|
| Dihydrotetrabenazine isomer | $^{1}$H-NMR spectrum ($CDCl_3$) | $^{13}$C-NMR spectrum ($CDCl_3$) | IR Spectrum (KBr solid) | Mass Spectrum (ES$^{+}$) |
| Isomers A and B 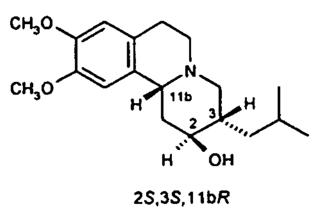 2*S*,3*S*,11b*R* | 6.675 1H (s);<br>6.57 δ 1H (s);<br>3.84 δ 6H (s);<br>3.55 δ 1H (br. d);<br>3.08 δ 1H (m);<br>2.79 δ 2H (m);<br>2.55 δ 3H (m);<br>2.17 δ 1H (m); . | 147.7δ;<br>147.6δ;<br>130.5δ;<br>127.6δ;<br>112.15;<br>108.4δ;<br>70.5δ;<br>57.5δ; | 2950 cm$^{-1}$;<br>2928 cm$^{-1}$;<br>2868 cm$^{-1}$;<br>2834 cm$^{-1}$;<br>1610 cm$^{-1}$;<br>1511 cm$^{-1}$;<br>1464 cm$^{-1}$<br>1364 cm$^{-1}$; | MH$^{+}$ 320 |

(continued)

Table 1

| Dihydrotetrabenazine isomer | $^{1}$H-NMR spectrum ($CDCl_3$) | $^{13}$C-NMR spectrum ($CDCl_3$) | IR Spectrum (KBr solid) | Mass Spectrum ($ES^+$) |
|---|---|---|---|---|
| OR<br>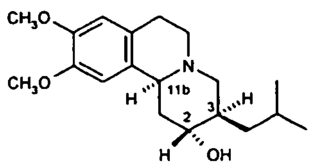<br>2*R*,3*R*,11b*S* | 1.72 δ 6H (m);<br>1.02 δ 1H (m);<br>0.88 δ 6H (t) | 56.5δ;<br>56.3δ;<br>54.8δ;<br>53.2δ;<br>40.4δ;<br>40.1δ;<br>36.0δ;<br>28.8δ;<br>26.2 δ;<br>23.7δ;<br>22.9 δ | 1324 $cm^{-1}$;<br>1258 $cm^{-1}$;<br>1223 $cm^{-1}$;<br>1208 $cm^{-1}$;<br>1144 $cm^{-1}$;<br>1045 $cm^{-1}$;<br>1006 $cm^{-1}$;<br>870 $cm^{-1}$;<br>785 $cm^{-1}$;<br>764 $cm^{-1}$; | |

Table 2

| Dihydrotetrabenazine isomer | $^{1}$H-NMR spectrum ($CDCl_3$) | $^{13}$C-NMR spectrum ($CDCl_3$) | IR Spectrum (KBr solid) | Mass Spectrum ($ES^+$) |
|---|---|---|---|---|
| Isomers C and D<br>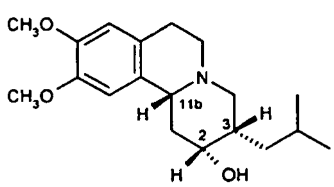<br>2*R*,3*S*,11b*R*<br>OR<br>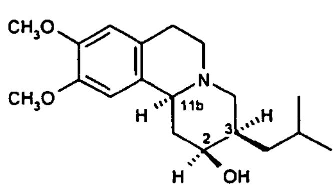<br>2*S*,3*R*,11b*S* | 6.68 δ 1H (s);<br>6.58 δ 1H (s);<br>3.92 δ 1H (m);<br>3.84 δ 6H (s);<br>3.15 δ 1H (m);<br>2.87 δ 3H(m);<br>2.43 δ 4H (m);<br>1.81 δ 1H (m);<br>1.64 δ 4H (m);<br>1.21 δ 1H (m);<br>0.94 δ 3H (d);<br>0.89 δ 3H (d) | 147.8 δ;<br>147.7 δ;<br>130.4 δ;<br>127.2 δ;<br>112.0 δ;<br>108.3 δ;<br>72.4 δ;<br>61.2 δ;<br>58.3 δ;<br>56.5 δ;<br>56.3 δ;<br>52.7 δ;<br>38.6 δ;<br>36.7 δ;<br>34.4 δ;<br>29.6 δ;<br>26.5 δ;<br>24.4 δ;<br>22.5 δ | 3370 $cm^{-1}$;<br>2950 $cm^{-1}$;<br>2929 $cm^{-1}$;<br>1611 $cm^{-1}$;<br>1512 $cm^{-1}$;<br>1463 cm-1<br>1362 $cm^{-1}$;<br>1334 $cm^{-1}$;<br>1259 $cm^{-1}$;<br>1227 $cm^{-1}$;<br>1148 $cm^{-1}$;<br>1063 $cm^{-1}$;<br>1024 $cm^{-1}$;<br>855 $cm^{-1}$;<br>766 $cm^{-1}$; | $MH^+$ 320 |

Tables 3 and 4 - Chromatography and ORD Data

| Table 3 | | |
|---|---|---|
| Dihydrotetrabenazine isomer | Chiral HPLC Methods and Retention Times | ORD (MeOH, 21˚C) |
| Isomers A and B<br>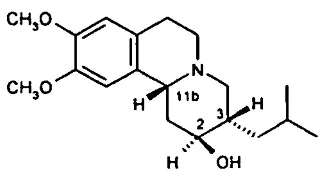<br>2*S*,3*S*,11b*R*<br>OR<br>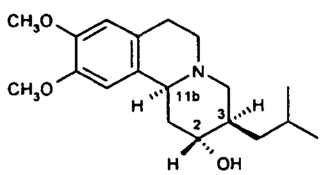<br>2*R*,3*R*,11b*S* | Column:<br>Chirex (S)-VAL, (R)-NEA, 250 x 4.6 mm<br>Mobile phase: Hexane : 1,2-dichloroethane : ethanol (36:62:2)<br>Flow: 1.0 ml $min^{-1}$<br>UV: 254 nm<br><br>Retention times:<br>Isomer A 16.6 min<br>Isomer B 15.3 min | Isomer A<br>[$\alpha_D$]-114.6˚<br><br>Isomer B<br>[$\alpha_D$] +123˚ |

| Table 4 | | |
|---|---|---|
| Isomers C and D<br>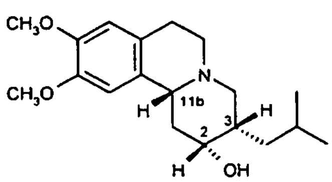<br>2*R*,3*S*,11b*R*<br>OR<br>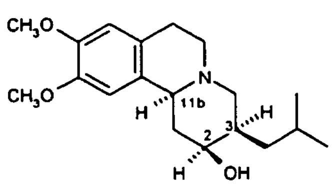<br>2*S*,3*R*,11b*S* | Column:<br>Chirex (S)-VAL, (R)-NEA, 250 x 4.6mm<br>Mobile phase: Hexane : ethanol (92:8)<br>Flow: 1.0 ml $min^{-1}$<br>UV: 254 nm<br><br>Retention times:<br>Isomer C 20.3 min<br>Isomer D 19.4 min | Isomer C<br>[$\alpha_D$] +150.9˚<br><br>Isomer D<br>[$\alpha_D$] -145.7˚ |

EXAMPLE 3

Alternative Method of Preparation of Isomer B and Preparation of Mesylate Salt

3A. Reduction of *RR*/*SS* Tetrabenazine

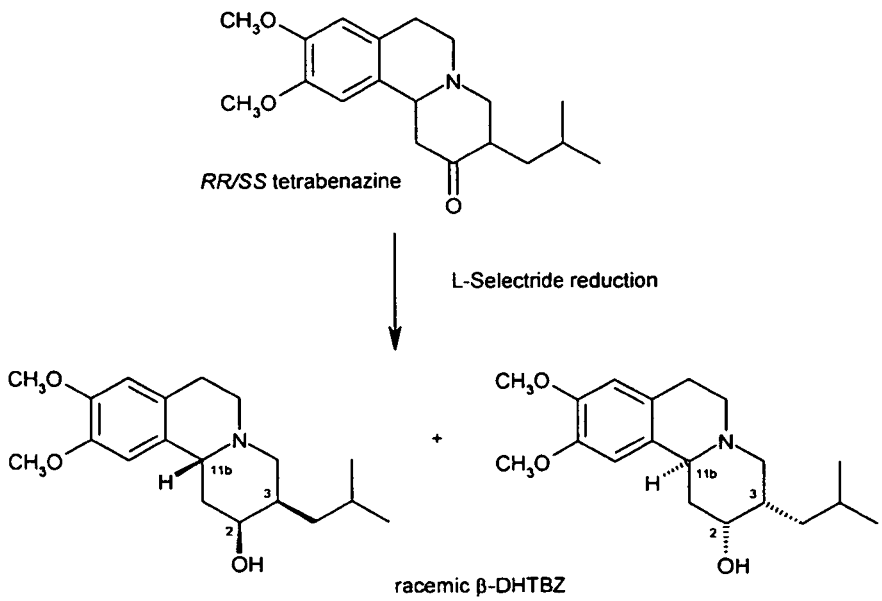


*RR/SS* tetrabenazine

racemic β-DHTBZ

2*S*, 3*R*, 11b*R* 2*R*, 3*S*, 11b*S*

1M L-Selectride® in tetrahydrofuran (52 ml, 52 mmol, 1.1 eq) was added slowly over 30 minutes to a cooled (ice bath), stirred solution of tetrabenazine racemate (15 g, 47 mmol) in tetrahydrofuran (56 ml). After the addition was complete, the mixture was allowed to warm to room temperature and stirred for a further six hours. TLC analysis (silica, ethyl acetate) showed only very minor amounts of starting material remained.

The mixture was poured on to a stirred mixture of crushed ice (112 g), water (56 ml) and glacial acetic acid (12.2 g). The resulting yellow solution was washed with ether (2 x 50 ml) and basified by the slow addition of solid sodium carbonate (ca. 13 g). Pet-ether (30-40 ˚C) (56 ml) was added to the mixture with stirring and the crude β-DHTBZ was collected as a white solid by filtration.

The crude solid was dissolved in dichloromethane (ca. 150 ml) and the resulting solution washed with water (40 ml), dried using anhydrous magnesium sulphate, filtered and concentrated at reduced pressure to ca. 40 ml. A thick suspension of white solid was formed. Pet-ether (30-40 ˚C) (56 ml) was added and the suspension was stirred for fifteen minutes at laboratory temperature. The product was collected by filtration and washed on the filter until snow-white using pet-ether (30-40˚C) (40 to 60 ml) before air-drying at room temperature to yield β-DHTBZ (10.1 g, 67%) as a white solid. TLC analysis (silica, ethyl acetate) showed only one component.

3B. Preparation and Fractional Crystallisation of the Camphorsulphonic acid Salt of Racemic β-DHTBZ

The product of Example 3A and 1 equivalent of (*S*)-(+)-Camphor-10-sulphonic acid were dissolved with heating in the minimum amount of methanol. The resulting solution was allowed to cool and then diluted slowly with ether until formation of the resulting solid precipitation was complete. The resulting white crystalline solid was collected by filtration and washed with ether before drying.

The camphorsulphonic acid salt of (10 g) was dissolved in a mixture of hot absolute ethanol (170 ml) and methanol (30 ml). The resulting solution was stirred and allowed to cool. After two hours the precipitate formed was collected by filtration as a white crystalline solid (2.9 g). A sample of the crystalline material was shaken in a separating funnel with excess saturated aqueous sodium carbonate and dichloromethane. The organic phase was separated, dried over anhydrous magnesium sulphate, filtered and concentrated at reduced pressure. The residue was triturated using pet-ether (30-40 ˚C) and the organic solution concentrated once more. Chiral HPLC analysis of the salt using a Chirex (S)-VAL and (R)-NEA 250 x 4.6 mm column, and a hexane : ethanol (98:2) eluent at a flow rate of 1 ml/minute showed showed that the isolated β-DHTBZ was enriched in one enantiomer (e.e. ca. 80%).

The enriched camphorsulphonic acid salt (14 g) was dissolved in hot absolute ethanol (140 ml) and propan-2-ol (420 ml) was added. The resulting solution was stirred and a precipitate began to form within one minute. The mixture was allowed to cool to room temperature and stirred for one hour. The precipitate formed was collected by filtration, washed with ether and dried to give a white crystalline solid (12 g).

The crystalline material was shaken in a separating funnel with excess saturated aqueous sodium carbonate and dichloromethane. The organic phase was separated, dried over anhydrous magnesium sulphate, filtered and concentrated

at reduced pressure. The residue was triturated using pet-ether (30-40 ˚C) and the organic solution concentrated once more to yield (after drying in vacuo.) (+)-β-DHTBZ (6.6 g, ORD +107.8˚). The isolated enantiomer has e.e. >97%.

3C. Preparation of Isomer B

A solution of phosphorus pentachloride (4.5 g, 21.6 mmol, 1.05 eq) in dichloromethane (55 ml) was added steadily over ten minutes to a stirred, cooled (ice-water bath) solution of the product of Example 3B (6.6 g, 20.6 mmol) in dichloromethane (90 ml). When the addition was complete, the resulting yellow solution was stirred for a further ten minutes before pouring on to a rapidly stirred mixture of sodium carbonate (15 g) in water (90 ml) and crushed ice (90 g). The mixture was stirred for a further 10 minutes and transferred to a separating funnel.

Once the phases had separated, the brown dichloromethane layer was removed, dried over anhydrous magnesium sulphate, filtered and concentrated at reduced pressure to give the crude alkene intermediate as brown oil (ca. 6.7 g). TLC analysis (silica, ethyl acetate) showed that no (+)-β-DHTBZ remained in the crude product.

The crude alkene was taken up (dry nitrogen atmosphere) in anhydrous tetrahydrofuran (40 ml) and a solution of borane in THF (1 M solution, 2.5 eq, 52 ml) was added with stirring over fifteen minutes. The reaction mixture was then stirred at room temperature for two hours. TLC analysis (silica, ethyl acetate) showed that no alkene intermediate remained in the reaction mixture.

A solution of sodium hydroxide (3.7 g) in water (10 ml) was added to the stirring reaction mixture, followed by an aqueous solution of hydrogen peroxide (50%, ca. 7 ml) and the two-phase mixture formed was stirred at reflux for one hour. TLC analysis of the organic phase at this time (silica, ethyl acetate) showed the appearance of a product with Rf as expected for Isomer B. A characteristic nonpolar component was also seen.

The reaction mixture was allowed to cool to room temperature and was poured into a separating funnel. The upper organic layer was removed and concentrated under reduced pressure to remove the majority of THF. The residue was taken up in ether (stabilised (BHT), 75 ml), washed with water (40 ml), dried over anhydrous magnesium sulphate, filtered and concentrated under reduced pressure to give a pale yellow oil (8.1 g).

The yellow oil was purified using column chromatography (silica, ethyl acetate : hexane (80:20), increasing to 100% ethyl acetate) and the desired column fractions collected, combined and concentrated at reduced pressure to give a pale oil which was treated with ether (stabilised, 18 ml) and concentrated at reduced pressure to give Isomer B as a pale yellow solid foam (2.2 g).

Chiral HPLC using the conditions set out in Example 3B confirmed that Isomer B had been produced in an enantiomeric excess (e.e.) of greater than 97%.

The optical rotation was measured using a Bellingham Stanley ADP220 polarimeter and gave an [$\alpha_D$] of + 123.5˚.

3D. Preparation of the Mesylate salt of Isomer B

The methanesulphonate salt of Isomer B was prepared by dissolving a mixture of 1 equivalent of Isomer B from Example 3C and 1 equivalent of methane sulphonic acid in the minimum amount of ethanol and then adding diethyl ether. The resulting white precipitate that formed was collected by filtration and dried *in vacuo* to give the mesylate salt in a yield of ca. 85% and a purity (by HPLC) of ca. 96%.

EXAMPLE 4

X-Ray Crystallographic Studies on Isomer B

The (*S*)-(+)-Camphor-10-sulphonic acid salt of Isomer B was prepared and a single crystal was subjected to X-ray crystallographic studies under the following conditions:

Diffractometer: Nonius KappaCCD area detector (t/i scans and OJ scans to fill asymmetric unit).
Cell determination: DirAx (Duisenberg, A.J.M.( 1992). J Appl. Cryst. 25, 92-96.)
Data collection: Collect (Collect: Data collection software, R. Hooft, Nonius B. V, 1998)
Data reduction and cell refinement: Demo (Z. Otwinowski & W. Minor, Methods in Enzymology (1997) Vol. 276: Macromolecular Crystallography, part A, pp. 307-326; C. W. Carter, Jr & R. M. Sweet, Eds., Academic Press).
Absorption correction: Sheldrick, G. M. SADABS - Bruker Nonius area detector scaling and absorption correction - V2.\ 0
Structure solution: SHELXS97 (G. M. Sheldrick, Acta Cryst. (1990) A46 467-473). Structure refinement: SHELXL97 (G. M. Sheldrick (1997), University of Göttingen, Germany)
Graphics: Cameron - A Molecular Graphics Package (D. M. Watkin, L. Pearce and C. K. Prout, Chemical Crystallography Laboratory, University of Oxford, 1993)

Special details: All hydrogen atoms were placed in idealised positions and refined using a riding model, except those of the NH and OH which were located in the difference map and refined using restraints. Chirality: NI=R, CI2=S, CI3=S, CI5=R, C21=S, C24=R

The results of the studies are set out below in Tables A, B, C, D and E.

In the Tables, the label RUS0350 refers to Isomer B.

**TABLE A**

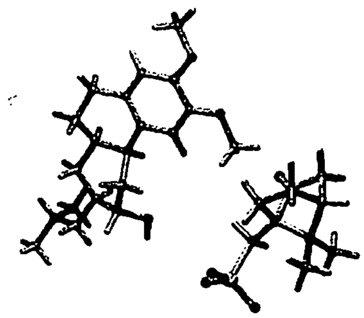

| | |
|---|---|
| Identification code | **2005bdy0585 (RUS0350)** |
| Empirical formula | $C_{29}H_{45}NO_7S$ |
| Formula weight | 551.72 |
| Temperature | 120(2) K |
| Wavelength | 0.71073 Å |
| Crystal system | Orthorhombic |
| Space group | $P2_12_12_1$ |
| Unit cell dimensions | $\alpha$ = 7.1732(9) Å |
| | *b* = 12.941 (2) Å |
| | *c* = 31.025 (4) Å |
| Volume | 2880.1(7) $Å^3$ |
| Z | 4 |
| Density (calculated) | 1.272 Mg / $m^3$ |
| Absorption coefficient | 0.158 $mm^{-1}$ |
| *F(000)* | 1192 |
| Crystal | Colourless Slab |
| Crystal size | 0.2 x 0.2 x 0.04 $mm^3$ |
| $\theta$ range for data collection | 3.06 - 27.37° |
| Index ranges | $-8 \leq h \leq 9, -16 \leq k \leq 16, -36 \leq l \leq 39$ |
| Reflections collected | 36802 |
| Independent reflections | 6326 [$R_{int}$ = 0.0863] |
| Completeness to $\theta$= 27.37° | 97.1 % |
| Absorption correction | Semi-empirical from equivalents |
| Max. and min. transmission | 0.9937 and 0.9690 |
| Refinement method | Full-matrix least-squares on $F^2$ |
| Data / restraints / parameters | 6326 / 1 / 357 |
| Goodness-of-fit on $F^2$ | 1.042 |
| Final *R* indices [$F^2 > 2\sigma(F^2)$] | *R1* = 0.0498, *wR2* = 0.0967 |
| *R* indices (all data) | *R1* = 0.0901, *wR2* = 0.1108 |
| Absolute structure parameter | 0.04(8) |
| Extinction coefficient | 0.0059(7) |
| Largest diff. peak and hole | 0.236 and -0.336 e $A^{-3}$ |

TABLE B. Atomic coordinates [x $10^4$], equivalent isotropic displacement parameters [$A^2$ x $10^3$] and site occupancy factors. $U_{eq}$ is defined as one third of the trace of the orthogonalized $U^{ij}$ tensor.

| Atom | *x* | *y* | *z* | *Ueq* | *S.o.f* |
|---|---|---|---|---|---|
| NI | 4839(3) | 11119(2) | 2180(1) | 24(1) | 1 |
| 01 | 2515(3) | 13171(1) | 349(1) | 31(1) | 1 |
| 02 | 5581 (3) | 14030(1) | 598(1) | 32(1) | 1 |
| 03 | 9220(3) | 12834(2) | 2385(1) | 36(1) | 1 |
| CI | 870(4) | 12674(2) | 190(1) | 36(1) | 1 |
| C2 | 3176(3) | 12838(2) | 739(1) | 25(1) | 1 |
| C3 | 2346(4) | 12109(2) | 997(1) | 25(1) | 1 |

(continued)

| Atom | x | y | z | Ueq | S.o.f |
|---|---|---|---|---|---|
| C4 | 3124(3) | 11821 (2) | 1395(1) | 24(1) | 1 |
| C5 | 4773(3) | 12276(2) | 1527(1) | 23(1) | 1 |
| C6 | 5629(4) | 13024(2) | 1262(1) | 24(1) | 1 |
| C7 | 4861(4) | 13308(2) | 875(1) | 25(1) | 1 |
| C8 | 7189(4) | 14582(2) | 747(1) | 38(1) | 1 |
| C9 | 2182(3) | 11023(2) | 1673(1) | 28(1) | 1 |
| C10 | 2759(3) | 11118(2) | 2137(1) | 26(1) | 1 |
| C11 | 5366(3) | 11096(2) | 2656(1) | 25(1) | 1 |
| C12 | 7292(4) | 11536(2) | 2747(1) | 25(1) | 1 |
| C13 | 7468(4) | 12663(2) | 2590(1) | 25(1) | 1 |
| C14 | 5988(4) | 12911(2) | 2252(1) | 25(1) | 1 |
| C15 | 5773(4) | 12010(2) | 1943(1) | 24(1) | 1 |
| C16 | 7734(4) | 11477(2) | 3232(1) | 28(1) | 1 |
| C17 | 7752(4) | 10418(2) | 3449(1) | 34(1) | 1 |
| C18 | 9198(6) | 9696(3) | 3249(1) | 65(1) | 1 |
| C19 | 8114(4) | 10562(2) | 3930(1) | 41(1) | 1 |
| C20 | 7509(4) | 8131(2) | 1250(1) | 31(1) | 1 |
| S1 | 7409(1) | 8792(1) | 1754(1) | 27(1) | 1 |
| 04 | 7758(2) | 7965(1) | 2064(1) | 30(1) | 1 |
| 05 | 8831(3) | 9582(2) | 1760(1) | 49(1) | 1 |
| 06 | 5524(2) | 9221(1) | 1798(1) | 32(1) | 1 |
| 07 | 7406(3) | 6932(1) | 498(1) | 48(1) | 1 |
| C21 | 6858(3) | 8622(2) | 830(1) | 25(1) | 1 |
| C22 | 7154(4) | 7851(2) | 459(1) | 30(1) | 1 |
| C23 | 7073(4) | 8450(2) | 40(1) | 32(1) | 1 |
| C24 | 6648(3) | 9544(2) | 203(1) | 28(1) | 1 |
| C25 | 4742(3) | 8877(2) | 787(1) | 29(1) | 1 |
| C26 | 4742(3) | 8877(2) | 787(1) | 29(1) | 1 |
| C27 | 7773(4) | 9610(2) | 630(1) | 25(1) | 1 |
| C28 | 7431(4) | 10628(2) | 868(1) | 29(1) | 1 |
| C29 | 9895(4) | 9489(2) | 569(1) | 36(1) | 1 |

TABLE C. Bond lengths [A] and angles [˚].

| | | | |
|---|---|---|---|
| NI-ClO | 1.498(3) | C14-C15 | 1.518(3) |
| NI-Cl5 | 1.522(3) | C16-C17 | 1.526(3) |
| NI-Cll | 1.524(3) | C17-C18 | 1.527(4) |
| 01-C2 | 1.368(3) | C17-C19 | 1.527(4) |
| Ol-Cl | 1.432(3) | C20-C21 | 1.525(3) |
| 02-C7 | 1.369(3) | C20-S I | 1.784(2) |
| 02-C8 | 1.433(3) | SI-05 | 1.4442(19) |
| 03-C13 | 1.425(3) | SI-04 | 1.4607(17) |
| C2-C3 | 1.372(3) | SI-06 | 1.4676(18) |
| C2-C7 | 1.417(3) | 07-C22 | 1.208(3) |
| C3-C4 | 1.407(3) | C21-C22 | 1.537(4) |
| C4-C5 | 1.384(3) | C21-C26 | 1.559(3) |
| C4-C9 | 1.506(3) | C21-C27 | 1.565(3) |
| C5-C6 | 1.411(3) | C22-C23 | 1.517(4) |
| C5-C15 | 1.516(3) | C23-C24 | 1.535(4) |

(continued)

| | | | |
|---|---|---|---|
| C6-C7 | 1.372(3) | C24-C25 | 1.548(4) |
| C9-C10 | 1.504(3) | C24-C27 | 1.554(4) |
| Cll-Cl2 | 1.521 (3) | C25-C26 | 1.557(4) |
| C12-C16 | 1.540(3) | C27-C28 | 1.529(3) |
| C12-C13 | 1.544(3) | C27-C29 | 1.542(4) |
| C13-C14 | 1.524(3) | | |
| Cl0-Nl-Cl5 | 113.33(19) | Cl2-Cll-Nl | 113.43(19) |
| Cl0-Nl-Cll | 109.46(18) | Cll-Cl2-Cl6 | 110.5(2) |
| Cl5-Nl-Cll | 111.96(19) | Cll-Cl2-Cl3 | 111.7(2) |
| C2-01-Cl | 116.6(2) | Cl6-Cl2-Cl3 | 109.84(19) |
| C7-02-C8 | 116.27(19) | 03-Cl3-Cl4 | 106.0(2) |
| 01-C2-C3 | 125.5(2) | 03-Cl3-Cl2 | 111.1 (2) |
| 01-C2-C7 | 115.0(2) | Cl4-Cl3-Cl2 | 111.0(2) |
| C3-C2-C7 | 119.5(2) | Cl5-Cl4-Cl3 | 110.1 (2) |
| C2-C3-C4 | 121.5(2) | C5-Cl5-Cl4 | 114.3(2) |
| C5-C4-C3 | 119.2(2) | C5-Cl5-Nl | 112.0(2) |
| C5-C4-C9 | 120.3(2) | Cl4-Cl5-Nl | 108.7(2) |
| C3-C4-C9 | 120.5(2) | Cl7-Cl6-Cl2 | 118.4(2) |
| C4-C5-C6 | 119.4(2) | Cl6-Cl7-Cl8 | 112.2(2) |
| C4-C5-Cl5 | 124.1(2) | Cl6-Cl7-Cl9 | 108.7(2) |
| C6-C5-Cl5 | 116.6(2) | Cl8-Cl7-Cl9 | 110.8(3) |
| C7-C6-C5 | 121.3(2) | C21-C20-S1 | 122.51 (18) |
| 02-C7-C6 | 125.4(2) | 05-Sl-04 | 112.93(11) |
| 02-C7-C2 | 115.4(2) | 05-Sl-06 | 112.47(12) |
| C6-C7-C2 | 119.2(2) | 04-Sl-06 | 111.93(11) |
| Cl0-C9-C4 | 111.7(2) | 05-Sl-C20 | 108.81(13) |
| Nl-Cl0-C9 | 111.0(2) | 04-Sl-C20 | 102.60(11) |
| 06-Sl-C20 | 107.44(12) | C23-C24-C25 | 106.4(2) |
| C20-C21-C22 | 109.0(2) | C23-C24-C27 | 103.3(2) |
| C20-C21-C26 | 117.3(2) | C25-C24-C27 | 102.3(2) |
| C22-C21-C26 | 102.1(2) | C24-C25-C26 | 102.9(2) |
| C20-C21-C27 | 123.4(2) | C25-C26-C21 | 104.2(2) |
| C22-C21-C27 | 100.21(19) | C28-C27-C29 | 107.8(2) |
| C26-C21-C27 | 101.7(2) | C28-C27-C24 | 112.0(2) |
| 07-C22-C23 | 126.4(2) | C29-C27-C24 | 113.7(2) |
| 07-C22-C21 | 125.9(2) | C28-C27-C21 | 116.5(2) |
| C23-C22-C21 | 107.7(2) | C29-C27-C21 | 112.3(2) |
| C22-C23-C24 | 101.3(2) | C24-C27-C21 | 94.27(19) |

TABLE D. Anisotropic displacement parameters [$A^2$x $10^3$]. The anisotropic displacement factor exponent takes the form:- $2\pi^2[h^2a^{*2}U^{11}+ ... + 2 h k a^* b^* U^{12}]$.

| *Atom* | $U_{ll}$ | $U_{22}$ | $U_{33}$ | $U_{23}$ | $U_{l3}$ | $U_{12}$ |
|---|---|---|---|---|---|---|
| NI | 26(1) | 24(1) | 23(1) | 2(1) | -1(1) | -3(1) |
| 01 | 37(1) | 30(1) | 24(1) | 3(1) | -7(1) | -4(1) |
| 02 | 41(1) | 31(1) | 25(1) | 5(1) | -2(1) | -10(1) |
| 03 | 26(1) | 49(1) | 32(1) | 7(1) | -3(1) | -9(1) |
| CI | 41(2) | 36(2) | 32(2) | 3(1) | -9(1) | -8(2) |
| C2 | 30(2) | 24(2) | 22(1) | 1(1) | -1(1) | 2(1) |

(continued)

| Atom | $U_{ll}$ | $U_{22}$ | $U_{33}$ | $U_{23}$ | $U_{l3}$ | $U_{12}$ |
|---|---|---|---|---|---|---|
| C3 | 25(1) | 26(1) | 24(1) | -3(1) | -2(1) | 2(1) |
| C4 | 26(2) | 22(1) | 23(1) | -1(1) | 2(1) | -1(1) |
| C5 | 24(1) | 22(1) | 23(1) | -2(1) | 1(1) | 0(1) |
| C6 | 26(1) | 22(1) | 24(1) | -3(1) | 2(1) | -5(1) |
| C7 | 30(2) | 22(1) | 22(1) | 2(1) | 4(1) | -4(1) |
| C8 | 45(2) | 34(2) | 36(2) | 5(1) | -2(1) | -20(2) |
| C9 | 23(1) | 32(1) | 29(2) | 3(1) | -1(1) | -4(1) |
| ClO | 26(1) | 29(1) | 25(1) | 2(1) | 0(1) | -5(1) |
| C11 | 31(1) | 25(1) | 20(1) | 2(1) | 0(1) | -2(1) |
| C12 | 26(1) | 26(1) | 23(1) | -1(1) | 1(1) | -1(1) |
| Cl3 | 26(1) | 28(1) | 23(1) | -1(1) | -1(1) | -2(1) |
| Cl4 | 30(2) | 22(2) | 24(1) | -1(1) | 1(1) | -1(1) |
| Cl5 | 22(1) | 22(1) | 28(1) | 2(1) | 0(1) | -4(1) |
| C16 | 31(1) | 28(1) | 24(1) | -1(1) | -3(1) | 3(1) |
| Cl7 | 46(2) | 31(2) | 25(1) | 1(1) | -7(1) | 0(2) |
| Cl8 | 106(3) | 46(2) | 41 (2) | 6(2) | -1(2) | 31 (2) |
| C19 | 51(2) | 41(2) | 31(2) | 9(2) | -7(1) | -4(2) |
| C20 | 30(2) | 34(2) | 29(1) | 2(1) | 3(1) | 9(2) |
| S1 | 27(1) | 30(1) | 24(1) | 4(1) | -2(1) | -5(1) |
| 04 | 31(1) | 36(1) | 23(1) | 9(1) | -1(1) | 0(1) |
| 05 | 53(1) | 58(1) | 37(1) | 13(1) | -11(1) | -35(1) |
| 06 | 34(1) | 35(1) | 28(1) | -3(1) | -2(1) | 10(1) |
| 07 | 81(2) | 25(1) | 40(1) | -1(1) | 12(1) | 6(1) |
| C21 | 26(1) | 25(2) | 24(1) | -1(1) | 3(1) | 2(1) |
| C22 | 35(2) | 25(2) | 31(2) | 0(1) | 1(1) | -1(1) |
| C23 | 40(2) | 30(2) | 25(1) | -2(1) | 1(1) | -2(1) |
| C24 | 28(1) | 29(2) | 26(2) | 2(1) | 2(1) | 2(1) |
| C25 | 30(2) | 34(2) | 29(2) | -1(1) | -2(1) | 0(1) |
| C26 | 26(1) | 34(2) | 28(2) | 0(1) | 1(1) | -5(1) |
| C27 | 23(1) | 26(1) | 26(1) | 0(1) | 2(1) | 0(1) |
| C28 | 31(1) | 26(1) | 30(1) | 0(1) | -2(1) | -6(1) |
| C29 | 29(2) | 41(2) | 40(2) | 0(2) | 2(1) | -3(1) |

TABLE E. Hydrogen coordinates [x $10^4$] and isotropic displacement parameters [$A^2$ x $10^3$].

| Atom | x | y | z | $U_{eq}$ | S.o.f |
|---|---|---|---|---|---|
| H98 | 5190(40) | 10528(15) | 2062(10) | 70(8) | 1 |
| H99 | 10030(50) | 12950(30) | 2575(12) | 70(8) | 1 |
| H1A | 1107 | 11933 | 156 | 54 | 1 |
| H1B | 529 | 12973 | -89 | 54 | 1 |
| H1C | -154 | 12777 | 395 | 54 | 1 |
| H3 | 1220 | 11793 | 904 | 30 | 1 |
| H6 | 6760 | 13337 | 1353 | 29 | 1 |
| H8A | 6872 | 14966 | 1009 | 58 | 1 |
| H8B | 7600 | 15065 | 523 | 58 | 1 |
| H8C | 8193 | 14091 | 810 | 58 | 1 |
| H9A | 814 | 11106 | 1651 | 33 | 1 |
| H9B | 2505 | 10324 | 1567 | 33 | 1 |
| H10A | 2250 | 11767 | 2259 | 32 | 1 |
| H10B | 2235 | 10534 | 2304 | 32 | 1 |

(continued)

| Atom | x | y | z | $U_{eq}$ | S.o.f |
|---|---|---|---|---|---|
| H11A | 4431 | 11494 | 2822 | 30 | 1 |
| H11B | 5322 | 10372 | 2759 | 30 | 1 |
| H12 | 8230 | 11108 | 2589 | 30 | 1 |
| H13 | 7334 | 13145 | 2840 | 30 | 1 |
| H14A | 4783 | 13050 | 2397 | 30 | 1 |
| H14B | 6354 | 13538 | 2090 | 30 | 1 |
| H15 | 7056 | 11776 | 1864 | 29 | 1 |
| H16A | 8973 | 11796 | 3278 | 33 | 1 |
| H16B | 6813 | 11911 | 3386 | 33 | 1 |
| I H17 | 6493 | 10098 | 3412 | 41 | 1 |
| H18A | 8906 | 9588 | 2944 | 97 | 1 |
| H18B | 9176 | 9031 | 3400 | 97 | 1 |
| H18C | 10440 | 10005 | 3276 | 97 | 1 |
| H19A | 9329 | 10894 | 3971 | 62 | 1 |
| H19B | 8110 | 9887 | 4073 | 62 | 1 |
| H19C | 7135 | 10999 | 4054 | 62 | 1 |
| H20A | 8824 | 7924 | 1207 | 37 | 1 |
| H20B | 6787 | 7484 | 1286 | 37 | 1 |
| H23A | 6070 | 8190 | -151 | 38 | 1 |
| H23B | 8277 | 8423 | -116 | 38 | 1 |
| H24 | 6928 | 10107 | -8 | 33 | 1 |
| H25A | 3773 | 9195 | 153 | 37 | 1 |
| H25B | 4152 | 10235 | 426 | 37 | 1 |
| H26A | 3994 | 8237 | 764 | 35 | 1 |
| H26B | 4300 | 9279 | 1039 | 35 | 1 |
| H28A | 8160 | 10638 | 1135 | 44 | 1 |
| I H28B | 6103 | 10692 | 936 | 44 | 1 |
| H28C | 7811 | 11207 | 684 | 44 | 1 |
| H29A | 10358 | 10042 | 381 | 54 | 1 |
| H29B | 10159 | 8817 | 436 | 54 | 1 |
| H29C | 10517 | 9531 | 849 | 54 | 1 |

**Table 6.** Hydrogen bonds [Å and °].

| *D*-H···*A* | *d*(*D*-H) | *d*(H···*A*) | *d*(*D*···*A*) | <(*DHA*) |
|---|---|---|---|---|
| N1-H98···O6 | 0.885(10) | 1.895(12) | 2.773(3) | 171(3) |
| N1-H98···S1 | 0.885(10) | 2.914(14) | 3.771(2) | 163(3) |
| 03-H99···$O4^{i}$ | 0.84(4) | 1.94(4) | 2.766(3) | 165(3) |
| 03-H99...$S1^{i}$ | 0.84(4) | 2.98(4) | 3.811(2) | 169(3) |
| Symmetry transformations used to generate equivalent atoms: (i) -x+2,y+1/2,-z+1/2 | | | | |

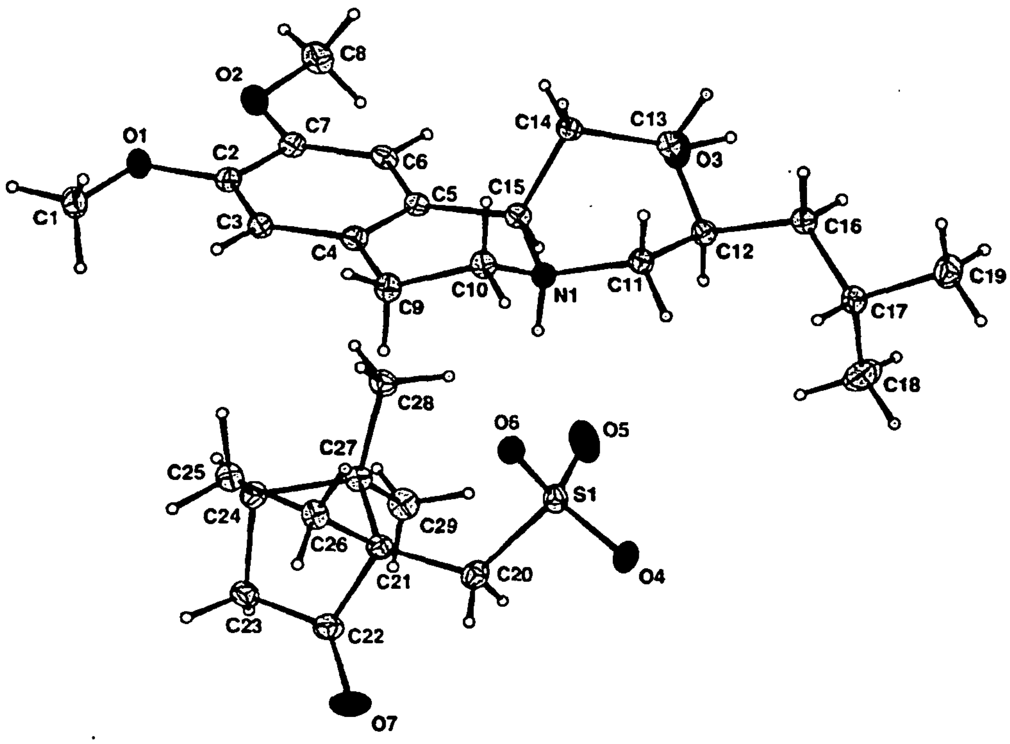

Thermal ellipsoids drawn at the 30% probability level

On the basis of the data set out above, Isomer B is believed to have the 2S, 3S, 11 bR configuration, which corresponds to Formula (Ia):

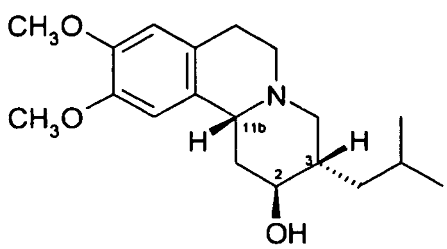

(Ia) – Isomer B

EXAMPLE 5

Receptor and Transporter Protein Binding Studies

The four dihydrotetrabenazine isomers A, B, C and D were subjected to specific binding assays to test their ability to bind to the receptors and transporter proteins described below. The results are set out in Table 5

**(a) Adrenergic $\alpha_{2A}$ Receptor:**

| | |
|---|---|
| Reference: | S. Uhlen et al. J Pharmacol. Exp. Ther., 271:1558-1565 (1994) |
| Source: | Human recombinant insect Sf9 cells |

(continued)

| | |
|---|---|
| Ligand: | 1 nM [$^3$H] MK-912 |
| Vehicle: | 1% DMSO |
| Incubation time/Temp: | 60 minutes @ 25 ˚C |
| Incubation buffer: | 75mM Tris-HCl, pH 7.4, 12.5mM $MgCl_2$, 2mM EDTA |
| Non Specific ligand: | 10μM WB-4101 |
| $K_d$: | 0.6 nM |
| $B_{max}$: | 4.6 pmole/mg protein |
| Specific binding: | 95% |
| Quantitation method: | Radioligand binding |
| Significance criteria: | ≥ 50% of maximum stimulation or inhibition |

**(b) Adrenergic $\alpha_{2B}$ Receptor:**

| | |
|---|---|
| Reference: | S. Uhlen et al., Eur. J. Pharmacol., 33 (1): 93-1-1 (1998) |
| Source: | Human recombinant CHO-K1 cells |
| Ligand: | 2.5 nM [3H] Rauwolscine |
| Vehicle: | 1% DMSO |
| Incubation time/Temp: | 60 minutes @ 25 ˚C |
| Incubation buffer: | 50 mM Tris-HCl, 1 mM EDTA, 12.5mM $MgCl_2$, pH |
| 7.4, | 0.2% BSA at 25 ˚C |
| Non Specific ligand: | 10 μM Prazosin |
| $K_d$: | 2.1 nM |
| $B_{max}$: | 2.1 pmole/mg protein |
| Specific binding: | 90% |
| Quantitation method: | Radioligand binding |
| Significance criteria: | ≥ 50% of maximum stimulation or inhibition |

**(c) Dopamine $D_1$ Receptor:**

| | |
|---|---|
| Reference: | Dearry et al., Nature, 347:72-76, (1990) |
| Source: | Human recombinant CHO cells |
| Ligand: | 1.4 nM [3H] SCH-23390 |
| Vehicle: | 1% DMSO |
| Incubation time/Temp: | 2 hours @ 37 ˚C |
| Incubation buffer: | 50 mM Tris-HCl, pH 7.4, 150 nM NaCl, 1.4 nM ascorbic acid, 0.001% BSA |
| Non Specific ligand: | 10 μM (+)-butaclamol |
| $K_d$: | 1.4 nM |
| $B_{max}$: | 0.63 pmole/mg protein |
| Specific binding: | 90% |
| Quantitation method: | Radioligand binding |
| Significance criteria: | ≥ 50% of maximum stimulation or inhibition |

**(d) Dopamine $D_{2L}$ Receptor:**

| | |
|---|---|
| Reference: | Bunzo et al., Nature, 336:783-787 (1988) |

(continued)

| | |
|---|---|
| Source: | Human recombinant CHO cells |
| Ligand: | 0.16 nM [3H] Spiperone |
| Vehicle: | 1% DMSO |
| Incubation time/Temp: | 2 hours @ 25 ˚C |
| Incubation buffer: | 50 mM Tris-HCl, pH 7.4, 150 nM NaCl, 1.4 nM ascorbic acid, 0.001% BSA |
| Non Specific ligand: | 10 μM Haloperidol |
| $K_d$: | 0.08 nM |
| $B_{max}$: | 0.48 pmole/mg protein |
| Specific binding: | 85% |
| Quantitation method: | Radioligand binding |
| Significance criteria: | ≥ 50% of maximum stimulation or inhibition |

**(e) Dopamine $D_3$ Receptor:**

| | |
|---|---|
| Reference: | Sokoloff et al., Nature, 347:146-151, (1990) |
| Source: | Human recombinant CHO cells |
| Ligand: | 0.7 nM [3H] Spiperone |
| Vehicle: | 1% DMSO |
| Incubation time/Temp: | 2 hours @ 37 ˚C |
| Incubation buffer: | 50 mM Tris-HCl, pH 7.4, 150 nM NaCl, 1.4 nM ascorbic acid, 0.001% BSA |
| Non Specific ligand: | 25 μM S(-)-Sulpiride |
| $K_d$: | 0.36 nM |
| $B_{max}$: | 1.1 pmole/mg protein |
| Specific binding: | 85% |
| Quantitation method: | Radioligand binding |
| Significance criteria: | ≥ 50% of maximum stimulation or inhibition |

**(f) Imidazoline $I_2$ (Central) Receptor:**

| | |
|---|---|
| Reference: | Brown et al., Brit. J Pharmacol., 99:803-809, (1990) |
| Source: | Wistar rat cerebral cortex |
| Ligand: | 2 nM [3H] Idazoxan |
| Vehicle: | 1% DMSO |
| Incubation time/Temp: | 30 minutes @ 25˚C |
| Incubation buffer: | 50 mM Tris-HCl, 0.5 mM EDTA, pH 7.4 at 25 ˚C |
| Non Specific ligand: | 1 μM Idazoxan |
| $K_d$: | 4 nM |
| $B_{max}$: | 0.14 pmole/mg protein |
| Specific binding: | 85% |
| Quantitation method: | Radioligand binding |
| Significance criteria: | ≥ 50% of maximum stimulation or inhibition |

**(g) Sigma $\sigma_1$ Receptor:**

| | |
|---|---|
| Reference: | Ganapathy et al., Pharmacol. Exp. Ther., 289:251-260, (1999) |

(continued)

| | |
|---|---|
| Source: | Human jurkat cells |
| Ligand: | 8 nM [3H] Haloperidol |
| Vehicle: | 1% DMSO |
| Incubation time/Temp: | 4 hours @ 25 ˚C |
| Incubation buffer: | 5 mM K2HPO4/KH2PO4 buffer pH 7.5 |
| Non Specific ligand: | 10 μM Haloperidol |
| $K_d$: | 5.8 nM |
| $B_{max}$: | 0.71 pmole/mg protein |
| Specific binding: | 80% |
| Quantitation method: | Radioligand binding |
| Significance criteria: | ≥ 50% of maximum stimulation or inhibition |

**(h) Sigma $\sigma_2$ Receptor:**

| | |
|---|---|
| Reference: | Hashimoto et al., Eur. J Pharmacol., 236:159-163, (1993) |
| Source: | Wistar rat brain |
| Ligand: | 3 nM [3H] Ifenprodil |
| Vehicle: | 1% DMSO |
| Incubation time/Temp: | 60 minutes @ 37 ˚C |
| Incubation buffer: | 50 mM Tris-HCl, pH 7.4 |
| Non Specific ligand: | 10 μM Ifenprodil |
| $K_d$: | 4.8 nM |
| $B_{max}$: | 1.3 pmole/mg protein |
| Specific binding: | 85% |
| Quantitation method: | Radioligand binding |
| Significance criteria: | ≥ 50% of maximum stimulation or inhibition |

**(i) Serotonin Transporter (SERT):**

| | |
|---|---|
| Reference: | Gu et al., J. Biol. Chem., 269(10):7124-7130, (1994) |
| Source: | Human recombinant HEK-293 cells |
| Ligand: | 0.15 nM [125I] RTI-55 |
| Vehicle: | 1% DMSO |
| Incubation time/Temp: | 3 hours @ 4 ˚C |
| Incubation buffer: | 100 mM NaCl, 50 mM Tris HCl, 1 μM Leupeptin, 10 μM PMSF, pH 7.4 |
| Non Specific ligand: | 10 μM Imipramine |
| $K_d$: | 0.17 nM |
| $B_{max}$: | 0.41 pmole/mg protein |
| Specific binding: | 95% |
| Quantitation method: | Radioligand binding |
| Significance criteria: | ≥ 50% of maximum stimulation or inhibition |

**(j) Dopamine Transporter (DAT):**

| | |
|---|---|
| Reference: | Giros et al., Trends Pharmacol. Sci., 14, 43-49 (1993) Gu et al., J. Biol. Chem., 269(10): 7124-7130 (1994) |
| Source: | Human recombinant CHO cells |
| Ligand: | 0.15 nM [$^{125}$I] RTI-55 |
| Vehicle: | 1% DMSO |
| Incubation time/Temp: | 3 hours @ 4 °C |
| Incubation buffer: | 100 mM NaCl, 50 mM Tris HCl, 1 μM Leupeptin, 10 μM PMSF, pH 7.4 |
| Non Specific ligand: | 10 μM Nomifensine |
| $K_d$: | 0.58 nM |
| $B_{max}$: | 0.047 pmole/mg protein |
| Specific binding: | 90% |
| Quantitation method: | Radioligand binding |
| Significance criteria: | ≥ 50% of maximum stimulation or inhibition |

**(k) $\alpha_{2c}$ adrenergic receptor**

$\alpha_{2c}$ Adrenergic receptor binding activity was determined using the method of Uhlen et al., J. Pharmacol. Exp. Ther. (1994), 271:1558-1565, and the following conditions:

| | |
|---|---|
| Source: | Human recombinant insect Sf9 cells |
| Ligand: | 1 nM [$^3$H] MK-912 |
| Vehicle: | 1% DMSO |
| Incubation time/Temp: | 60 minutes @ 25 °C |
| Incubation buffer: | 75mM Tris-HCl, pH 7.4,12.5mM $MgCl_2$, 2mM EDTA |
| Non Specific ligand: | 10μM WB-4101 |
| $K_d$: | 0.17 nM |
| $B_{max}$: | 6.8 pmole/mg protein |
| Specific binding: | 95% |
| Quantitation method: | Radioligand binding |
| Significance criteria: | ≥ 50% of maximum stimulation or inhibition |

**(1) Serotonin (5-hydroxytryptamine) 5-$HT_{2b}$ receptor**

5-$HT_{2b}$ receptor binding activity was determined using the method of Bonhaus et al., Br. J Pharmacol., (1995) 115:622-628, and the following conditions:

| | |
|---|---|
| Source: | Human recombinant CHO-K1 cells |
| Ligand: | 1.2 nM [$^3$H] lysergic acid diethylamide (LSD) |
| Vehicle: | 1% DMSO |
| Incubation time/Temp: | 60 minutes @ 37 °C |
| Incubation buffer: | 50 mM Tris-HCl, pH 7.4, 4 mM $CaCl_2$, 0.1 % ascorbic acid |
| Non Specific ligand: | 10 μM serotonin |
| $K_d$: | 2.1 nM |
| $B_{max}$: | 1.1 pmole/mg protein |
| Specific binding: | 80% |
| Quantitation method: | Radioligand binding |
| Significance criteria: | ≥ 50% of maximum stimulation or inhibition |

**(m) Serotonin (5-hydroxytryptamine) 5-$HT_6$ receptor**

5-$HT_6$ receptor binding activity was determined using the method of Monsma et al., Mol. Pharmacol., (1993),

43:320-327, and the following conditions:

| | |
|---|---|
| Source: | Human recombinant HeLa cells |
| Ligand: | 1.5 nM [$^3$H] lysergic acid diethylamide (LSD) |
| Vehicle: | 1% DMSO |
| Incubation time/Temp: | 2 hours @ 37 ˚C |
| Incubation buffer: | 50 mM Tris-HCl, pH 7.4, 150 mM NaCl, 2 mM ascorbic acid, 0.001% BSA |
| Non Specific ligand: | 5 μM serotonin |
| $K_d$: | 1.3 nM |
| $B_{max}$: | 1.7 pmole/mg protein |
| Specific binding: | 90% |
| Quantitation method: | Radioligand binding |
| Significance criteria: | ≥ 50% of maximum stimulation or inhibition |

Table 5

| Percentage Inhibition by 10 μM Solutions of Dihydrotetrabenazine isomers of Specific Binding at Receptor and Transporter Proteins ($IC_{50}$ value, where measured, is in parentheses) | | | | |
|---|---|---|---|---|
| Receptor/Protein | Isomer A | Isomer B | Isomer C | Isomer D |
| (a) $\alpha_{2A}$ Receptor | 86 | 12 | 13 | 87 |
| (b) $\alpha_{2B}$ Receptor | 44 | 14 | -7 | 50 |
| (c) $D_1$ Receptor | 78 | 1 | 6 | 38 |
| (d) $D_{2L}$ Receptor | 87 | 16 | -14 | 58 |
| (e) $D_3$ Receptor | 69 | 7 | 9 | 63 |
| (f) $I_2$ Receptor | 74 | 8 | 0 | 55 |
| (g) $\sigma_1$ Receptor | 48 | 82 | 59 | 82 |
| (h) $\sigma_2$ Receptor | 64 | 64 | 61 | 69 |
| (i) SERT | 19 | 86 (0.35) | 77(2.75) | 8 |
| (j) DAT | 3 | 4 | -2 | 2 |
| (k) $\sigma_{2c}$ receptor | 56 | -6 | 3 | 74 |
| (1) 5-$HT_{2b}$ receptor | 74 | 10 | 14 | 43 |
| (m) 5-$HT_6$ receptor | 51 | 10 | 10 | 41 |

On the basis of the binding data for Isomers A and D for the dopamine and serotonin receptors, and by analogy with the dopamine-serotonin binding profiles of known antipsychotic agents, it is envisaged that Isomers A and D will be useful in the treatment of psychosis, for example psychosis arising from or associated with schizophrenia.

EXAMPLE 6

Cognitive function and antipsychotics: An investigation into the efficacy of Isomer A to improve a cognitive deficit induced by sub-chronic PCP in the novel object recognition task

Cognition in Schizophrenia:

The major clinical unmet need in schizophrenia is the treatment of negative and cognitive symptoms, since even the latest generation of atypical antipsychotic drugs offers little improvement. Notably, a cognitive deficit in patients with schizophrenia is now recognised as a core part of the disorder, and is believed to have a significant bearing on the patient's recovery and re-integration into society.

There have been few attempts to model the cognitive disturbances in schizophrenia, although some of the

more recent, and arguably more valid animal models show cognitive deficits. The classical approaches used to provide animal models for the testing of potential antipsychotics have relied on the use of dopaminergic drugs, the limitations of which are increasingly being recognised. Administration of the glutamate/NMDA antagonist phencyclidine (PCP) has been considered to provide a better model of schizophrenia in that it can induce both negative symptoms as well as the positive symptoms associated with amphetamine psychosis (J. D. Jentsch and R. H. Roth, Neuropsychopharmacology (1999) 20(3): 201-225). This approach may have some pathological validity in that there is evidence of abnormalities of glutamatergic systems in the brain in schizophrenia; such changes include deficits in cortico-striatal innervation that may contribute to, if not underlie, cognitive dysfunction in the disease (Aparicio-Legarza et al., Neurosci. Lett. (1997) 232, 13-16). In addition, some PCP-induced behaviours are reversed by certain atypical, but not typical antipsychotics (Geyer et al., Brain Res. Bull. (1990) 25: 485-498.). This suggests a potential correlation with effects on negative or other symptoms that respond less well to the typical drugs.

The novel object recognition paradigm:

Certain pre-clinical tests allow the observation of relatively subtle cognitive deficits in the rat that resemble cognitive symptoms in subjects with schizophrenia. The cognitive deficits observed are seen in behaviours such as working memory deficits which may be measured by recognition tasks such as the novel object recognition (NOR) paradigm. A recognition memory task allows the comparison between presented stimuli and previously stored information. Ennaceur & Delacour, Behav. Brain Res. 31: 47-59 (1988) described the NOR test in rats which was based on the differential exploration of familiar and new objects. The NOR test is a non-rewarded, ethologically relevant paradigm based on the spontaneous exploratory behaviour of rats which measures working memory. Each session consists of two trials. In the first trial, the rats are exposed to two identical objects in an open field. During the second trial, rats are exposed to two dissimilar objects, one familiar object from the first trial and one new object. Object recognition in rats can be measured as the difference in time spent exploring the familiar and the new object.

Rats have been shown to spend more time exploring the new object. It was found that rats are able to discriminate between the familiar and the novel object when the inter-trial interval is between 1 minute and 1-5 hours, but not when it is greater than 24 hours, although this effect may be sex dependent in the rat (Sutcliffe et al, A preliminary investigation into the effects of gender on cognition in male and female rats using the novel object recognition paradigm. Presented at the 96th meeting of the Society for Endocrinology, 7-9th November 2005). The duration of each trial is also important as a preference for the novel object only lasts during the first 1 or 2 minutes, after which time preference diminishes as both objects become familiar and are explored equally.

(Grayson and Neill, J. Psychopharmacology 18: A55, 2004; and Proceedings of the BPS at http://wwwpA2online.org/vol 12issue4-abst077P.html. 2005) have demonstrated a selective deficit in this task induced by acute and sub-chronic treatment with PCP. The deficit is only observed in the retention phase of the task, suggesting a specific and relatively subtle cognitive impairment. Thus behaviour in the acquisition phase of the test (and locomotor activity) is unaffected by PCP treatment. The effects of PCP in this paradigm may represent a selective deficit in working memory which is known to be impaired in schizophrenia. J. C. Neill's group at the University of Bradford, United Kingdom, have found that the atypical antipsychotic drug clozapine, but not the classical antipsychotic, haloperidol, can ameliorate (and prevent, Idris et al. Soc. Neurosci. abstr. 67.15.2005) the cognitive deficit induced by sub-chronic PCP (2mg/kg ip twice daily for 7 days followed by 7 days drug-free period) in this paradigm. Haloperidol is known to be ineffective in the treatment of cognitive deficit symptoms in schizophrenia, and indeed may make them worse, while atypical antipsychotics can improve certain aspects of cognition in schizophrenia. Furthermore, Grayson *et al,* have recently demonstrated efficacy of risperidone to attenuate the sub-chronic PCP-induced deficit in this paradigm. Thus this test has some predictive validity for the treatment of cognitive symptoms of schizophrenia. The sub-chronic PCP-induced deficit has been shown to be robust and long-lasting in female rats, i.e. up to 5 months post-treatment.

Object of the Experiment

The abovementioned rodent model was used to assess the effects of the Isomer A on sub-chronic PCP-induced deficits in working memory using the novel object recognition (NOR) paradigm. The working hypothesis was that both acute and sub-chronic treatment with Isomer A will attenuate the selective working memory deficit induced by sub-chronic PCP as measured in the NOR test paradigm. Female rats were used in this paradigm as it has previously been found that males to be less sensitive to the deficit induced by PCP (Grayson and Neill, *idem*.) and females show more robust performance following increasing inter-trial intervals compared with male rats (Sutcliffe *et al*, *idem*).

Methods

The Novel Object Recognition Paradigm:

Habituation.

Rats are allowed to habituate to the empty test box and the behavioural test room environment for 1 hour on day 1. Prior to behavioural testing on day 2 rats are given a further 3 minutes habituation.

Behavioural testing.

Following the 3 minute habituation period, the rats are given two 3 minute trials (T1 and T2) which are separated by a 1 minute inter-trial interval in the home cage during which the objects are changed.

T1=Trial 1, the acquisition trial.

In this trial, the animals are allowed to explore two identical objects (A1 and A2) for 3 minutes.

T2=Trial 2, the retention trial.

In this trial, the animals explore a familiar object (A) from T1 and a novel object (B) for 3 minutes. The familiar object presented during T2 is a duplicate of the object presented in T1 in order to avoid any olfactory trails.

Object exploration.

The object exploration is defined by animals licking, sniffing or touching the object with the forepaws whilst sniffing, but not leaning against, turning around, standing or sitting on the object. The exploration time (s) of each object (A1, A2, A and B) in each trial are recorded using two stopwatches and the following factors are calculated:

- Total exploration time of both objects in the acquisition trial (s).
- Total exploration time of both objects in the retention trial (s).
- Habituation of exploratory activity. The LMA includes the exploration time, as measured by the number of lines crossed, for both the trials.
- Discrimination index, which is calculated as shown below;

$$\text{(time spent exploring novel object} - \text{time spent exploring familiar object)} \div \text{total time spent in exploring the objects}$$

Behaviour in all trials was recorded on video for subsequent blind scoring.

Subjects

50 female hooded-Lister rats (Harlan, UK) were used as subjects for these studies. Rats were housed in groups of 5 under standard laboratory conditions under a 12hr light:dark cycle, lights on at 0700 hr. All testing was carried out in the light phase. Food and water were freely provided. All experiments were conducted in accordance with the Animals Scientific Procedures Act, U.K. 1986 and were approved by the University of Bradford ethical review panel.

Drugs

Rats were randomly assigned to two treatment groups and treated with vehicle, n=10 (distilled water, ip) or PCP, n=40 (2 mg/kg, ip) twice daily for 7 days. Phencyclidine hydrochloride (PCP, Sigma, UK) was dissolved in distilled water. This was followed by a 7 day wash out period before the rats were tested. Isomer A was dissolved in distilled

water and administered via the oral route at doses of 3, 10 and 30 mg/kg, 30 minutes prior to testing. Risperidone (0.2 mg/kg) was prepared in distilled water and injected i.p. 30 minutes prior to testing. All drugs were administered in a volume of 1 ml/kg. All drug doses were calculated as base equivalent weight.

Statistical Analysis

All data are expressed as mean ± s.e.m (n=7-10 per group) and were analysed by a two way ANOVA (factors are; drug and exploration time of the two objects) with further analysis by a post-hoc student's t-test (time spent exploring objects) or Dunnett's t-test (LMA and DI).

Drug treatment

Groups of rats (n=7-10) were tested in the NOR paradigm as described above. Rats were tested for their performance in the task following sub-chronic treatment with PCP (2 mg/kg i.p. twice daily for 7 days followed by 7 days drug-free period) or vehicle followed by acute treatment with Isomer A, risperidone or vehicle. Rats were randomly assigned to the drug treatment groups and received vehicle or Isomer A (3.0, 10 and 30mg/kg) p.o. 30 minutes prior to behavioural testing.

Results

The results are shown in Figures 1 to 4.

Figure 1 illustrates the mean exploration time of identical objects in the acquisition phase-T1- following acute administration of Isomer A (3.0-30 mg/kg, p.o) and risperidone (Risp 0.2mg/kg, i.p) in sub-chronic PCP (2mg/kg, i.p twice daily for seven days) and vehicle treated rats.

Figure 2 illustrates the ability of acute Isomer A (3-30mg/kg, p.o) and risperidone (Risp 0.2mg/kg, i.p) to attenuate the effect of sub-chronic PCP on the exploration time (s) of a familiar object and a novel object in a 3 minute retention trial in female hL rats. Significant difference between time spent exploring the familiar and novel object *$P<0.05$-***$P<0.001$.

Figure 3 illustrates the effect of Isomer A (3-30 mg/kg, p.o.) and risperidone (Risp 0.2mg/kg, i.p.) on the effect of sub-chronic PCP (2mg/kg, i.p twice daily for seven days) treatment on the discrimination index (DI).

Figure 4 illustrates the effect of acute administration (3-30 mg/kg, p.o.) of Isomer A and risperidone (Risp 0.2mg/kg, i.p) in sub-chronically PCP treated rats on the total number of line crossings in the novel object recognition task (T1+T2). **$p<0.01$; significant reduction in number of line crossings compared with the vehicle control group.

Acute PCP (0.5-2.0mg/kg ip) and sub-chronic PCP (2mg/kg i.p. twice daily for 7 days followed by 7 days drug-free period) produce a selective cognitive deficit in the retention phase of the NOR task in female rats (Grayson and Neill, 2004; 2005a). The atypical antipsychotic agent clozapine (1-5mg/kg), but not haloperidol (0.05-0.075mg/kg) significantly improved (and prevented, Idris et al, 2005) the deficit induced by sub-chronic PCP in this paradigm (Grayson and Neill, 2005a). The present results add to this existing data and show that Isomer A also has efficacy to attenuate the sub-chronic PCP-induced deficit in a manner similar to the atypical antipsychotic, risperidone.

The effects of acute treatment with Isomer A were selective for the retention phase of the NOR task (Figure 2). Its effects are consistent with improvement of working memory deficits induced by PCP in a paradigm with some validity for the pathology of schizophrenia. This effect was significant at the highest dose of Isomer (30mg/kg). In contrast, Isomer A had no effect on exploration of two identical objects in the acquisition phase of the task, Figure 1. 30 mg/kg of Isomer A also had a significant effect to reduce locomotor activity in the test arena, Figure 4. This was shown as a reduction in the number of lines crossed in the novel object arena in T1 and T2. Observation of the behaviour of the rats suggested that they spent more time in object than environment exploration which reduced their overall activity score in the box. They did not appear sedated. Data shown in Figure 3 show that sub-chronic PCP treatment induced a reduction in the discrimination index, and that this was improved following 30 mg/kg of Isomer A and 0.2mg/kg of risperidone: however, none of these effects reached statistical significance.

The results set out herein suggest that Isomer A may have some therapeutic value in improvement of cognitive deficit symptoms of schizophrenia.

EXAMPLE 7

Pharmaceutical Compositions

(i) Tablet Formulation - I

A tablet composition containing the dihydrotetrabenazine of the invention is prepared by mixing 50 mg of the dihydrotetrabenazine with 197 mg of lactose (BP) as diluent, and 3 mg magnesium stearate as a lubricant and compressing to form a tablet in known manner.

(ii) Tablet Formulation - II

A tablet composition containing the dihydrotetrabenazine of the invention is prepared by mixing the compound (25 mg) with iron oxide, lactose, magnesium stearate, starch maize white and talc, and compressing to form a tablet in known manner.

(iii) Capsule Formulation

A capsule formulation is prepared by mixing 100 mg of the dihydrotetrabenazine of the invention with 100 mg lactose and filling the resulting mixture into standard opaque hard gelatin capsules.

**Equivalents**

It will readily be apparent that numerous modifications and alterations may be made to the specific embodiments of the invention described above without departing from the principles underlying the invention. All such modifications and alterations are intended to be embraced by this application.

**Claims**

1. A 3,11 b-*cis*-dihydrotetrabenazine of the formula (Ib):

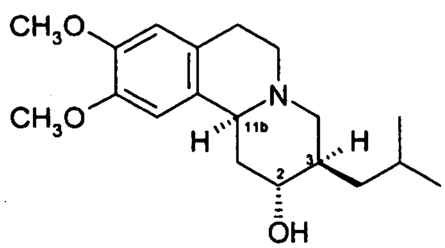

(Ib)

or a pharmaceutically acceptable salt thereof for use in the prophylaxis or treatment of psychosis.

2. A 3,11b-*cis*-dihydrotetrabenazine for use according to claim 1 wherein the use is the prevention or alleviation of a psychotic episode.

3. A 3,11b-*cis*-dihydrotetrabenazine for use according to claim 1 or claim 2 wherein the psychosis or psychotic episode arises from or is associated with schizophrenia.

4. A 3,11b-*cis*-dihydrotetrabenazine or a pharmaceutically acceptable salt thereof as defined in claim 1 for use in the prophylaxis or treatment of schizophrenia.

5. A 3,11b-*cis*-dihydrotetrabenazine for use according to any one of claims 1 to 3 wherein the psychotic episodes, psychoses or symptoms prevented or alleviated are selected from:

- delusions;

- hallucinations;
- visual hallucinations;
- auditory hallucinations;
- hallucinations involving tactile sensations, tastes or smells;
- confusion;
- emotional, behavioral, or intellectual disturbances;
- withdrawal from reality;
- illogical and/or disorganized patterns of thinking;
- paranoid or delusional beliefs;
- paranoia
- grandiose delusions;
- persecutory or self-blaming delusions; and
- personality changes.

**6.** A 3,11b-*cis*-dihydrotetrabenazine for use according to any one of claims 1 to 3 wherein the psychotic episodes, psychoses or symptoms prevented or alleviated are selected from those arising from or associated with:

- psychosis caused by or associated with schizophrenia;
- psychosis caused by or associated with bipolar disorder (manic depression);
- psychosis caused by or associated with severe clinical depression;
- psychosis induced by disorders and conditions such as:

    - o electrolyte disorder;
    - o urinary tract infections in the elderly;
    - o pain syndromes;
    - o drug toxicity;
    - o drug withdrawal; and
    - o infections of or injuries to the brain;

- psychosis caused by chronic psychological stress (brief reactive psychosis);
- psychosis triggered or exacerbated by severe mental stress; and
- psychosis triggered by or arising from or following illnesses and conditions such as AIDS, leprosy, malaria and mumps.

**7.** A 3,11b-*cis*-dihydrotetrabenazine for use according to claim 4 wherein the use is the alleviation or reduction of one or more symptoms of schizophrenia.

**8.** A 3,11b-*cis*-dihydrotetrabenazine for use according to claim 4 wherein the use is the prevention, alleviation or reduction of one or more symptoms selected from:

- delusions;
- hallucinations;
- confusion;
- emotional, behavioral, or intellectual disturbances;
- withdrawal from reality; and
- illogical patterns of thinking.

**9.** The 3,11b-*cis*-dihydrotetrabenazine for use according to any one of the preceding claims wherein the 3,11b-*cis*-dihydrotetrabenazine is in the form of an acid addition salt.

**10.** The 3,11b-*cis*-dihydrotetrabenazine for use according to claim 9 wherein the salt is a methane sulphonate salt.

**11.** The 3,11b-*cis*-dihydrotetrabenazine for use according to claim 1 wherein the use is the improvement of cognitive deficit symptoms of schizophrenia.

**12.** The use of a 3,11b-*cis*-dihydrotetrabenazine of the formula (Ib) as defined in any one of claims 1, 9 and 10 for the manufacture of a medicament for a therapeutic use as defined in any one of claims 1 to 8 and 11.

**Patentansprüche**

1. 3,11b-*cis*-Dihydrotetrabenazin der Formel (Ib):

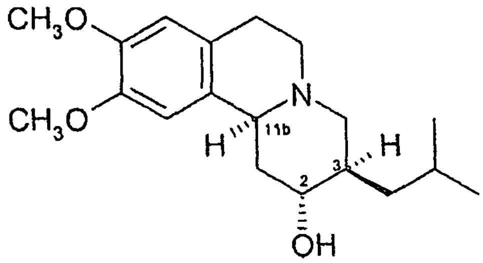

(Ib)

oder ein pharmazeutisch verträgliches Salz davon zur Anwendung in der Prophylaxe oder Behandlung von Psychose.

2. 3,11b-*cis*-Dihydrotetrabenazin zur Anwendung nach Anspruch 1, wobei die Anwendung zur Prävention oder Linderung einer psychotischen Episode vorgesehen ist.

3. 3,11b-*cis*-Dihydrotetrabenazin zur Anwendung nach Anspruch 1 oder 2, wobei die Psychose oder psychotische Episode aus der Schizophrenie entsteht oder mit ihr assoziiert ist.

4. 3,11b-*cis*-Dihydrotetrabenazin oder ein pharmazeutisch verträgliches Salz davon, wie nach Anspruch 1 definiert, zur Anwendung in der Prophylaxe oder Behandlung von Schizophrenie.

5. 3,11b-*cis*-Dihydrotetrabenazin zur Anwendung nach einem der Ansprüche 1 bis 3, wobei die verhinderten oder gelinderten psychotischen Episoden, Psychosen oder Symptome ausgewählt sind aus:

   - Wahnhaften Störungen;
   - Halluzinationen;
   - optischen Halluzinationen;
   - akustischen Halluzinationen;
   - Halluzinationen, an denen taktile Wahrnehmungen, Geschmack oder Geruch beteiligt sind;
   - Verwirrtheit;
   - emotionale, behaviorale oder intellektuelle Störungen;
   - Realitätsrückzug;
   - unlogische und/oder desorganisierte Denkmuster;
   - paranoide oder wahnhafte Überzeugungen;
   - Paranoia
   - Größenwahn;
   - Verfolgungs- oder Selbstbeschuldigungswahn; und
   - Persönlichkeitsänderungen.

6. 3,11b-*cis*-Dihydrotetrabenazin zur Anwendung nach einem der Ansprüche 1 bis 3, wobei die verhinderten oder gelinderten psychotischen Episoden, Psychosen oder Symptome aus denen ausgewählt sind, die aus Folgendem entstehen oder damit assoziiert sind:

   - Psychose, verursacht durch oder assoziiert mit Schizophrenie;
   - Psychose, verursacht durch oder assoziiert mit bipolarer Störung (manischer Depression);
   - Psychose, verursacht durch oder assoziiert mit schwerer klinischer Depression;
   - Psychose, induziert durch Störungen und Zustände, wie zum Beispiel:

     o Elektrolytstörung;
     o Harnwegsinfektionen bei älteren Menschen;
     o Schmerzsyndrome;

o Substanztoxizität;
o Substanzentzug; und
o Infektionen oder Verletzungen des Gehirns;

• Psychose, verursacht durch chronischen psychologischen Stress (kurze reaktive Psychose);
• Psychose, getriggert oder verschlimmert durch schweren mentalen Stress; und
• Psychose, getriggert durch oder entstanden aus oder nach Krankheiten und Zuständen, wie zum Beispiel AIDS, Lepra, Malaria und Mumps.

**7.** 3,11b-*cis*-Dihydrotetrabenazin zur Anwendung nach Anspruch 4, wobei die Anwendung zur Linderung oder Reduktion von einem oder mehr Symptom(en) der Schizophrenie vorgesehen ist.

**8.** 3,11b-*cis*-Dihydrotetrabenazin zur Anwendung nach Anspruch 4, wobei die Anwendung zur Prävention, Linderung oder Reduktion von einem oder mehr Symptom(en) vorgesehen ist, ausgewählt aus:

• Wahnhaften Störungen;
• Halluzinationen;
• Verwirrtheit;
• emotionalen, behavioralen oder intellektuellen Störungen;
• Realitätsrückzug; und
• unlogischen Denkmustern.

**9.** 3,11b-*cis*-Dihydrotetrabenazin zur Anwendung nach einem der vorangehenden Ansprüche, wobei das 3,11b-*cis*-Dihydrotetrabenazin in der Form eines Säureadditionssalzes vorliegt.

**10.** 3,11b-*cis*-Dihydrotetrabenazin zur Anwendung nach Anspruch 9, wobei das Salz ein Methansulfonatsalz darstellt.

**11.** 3,11b-*cis*-Dihydrotetrabenazin zur Anwendung nach Anspruch 1, wobei die Anwendung zur Besserung von kognitiven Defizitsymptomen der Schizophrenie vorgesehen ist.

**12.** Anwendung eines 3,11b-*cis*-Dihydrotetrabenazins der Formel (Ib), wie nach einem der Ansprüche 1, 9 und 10 definiert, zur Herstellung eines Arzneimittels zur therapeutischen Anwendung nach einem der Ansprüche 1 bis 8 und 11.

## Revendications

**1.** Une 3,11b-*cis*-dihydrotétrabénazine ayant pour formule (Ib) :

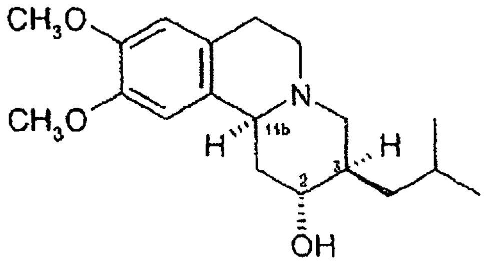

(Ib)

ou un sel pharmaceutiquement acceptable de celle-ci à utiliser dans la prophylaxie ou le traitement de la psychose.

**2.** Une 3,11b-*cis*-dihydrotétrabénazine à utiliser selon la revendication 1, dans laquelle l'utilisation est la prévention ou le soulagement d'un épisode psychotique.

**3.** Une 3,11b-*cis*-dihydrotétrabénazine à utiliser selon la revendication 1 ou la revendication 2, dans laquelle la psychose ou l'épisode psychotique découle de ou est associé à la schizophrénie.

**4.** Une 3,11b-*cis*-dihydrotétrabénazine ou un sel pharmaceutiquement acceptable de celle-ci, tel(le) que défini(e) dans la revendication 1, à utiliser dans la prophylaxie ou le traitement de la schizophrénie.

**5.** Une 3,11b-*cis*-dihydrotétrabénazine à utiliser selon l'une quelconque des revendications 1 à 3, dans laquelle les épisodes psychotiques, les psychoses ou les symptômes prévenus ou soulagés sont sélectionnés parmi :

- délire ;
- hallucinations ;
- hallucinations visuelles ;
- hallucinations auditives ;
- hallucinations impliquant des sensations tactiles, des goûts ou des odeurs ;
- confusion ;
- troubles émotionnels, comportementaux ou intellectuels ;
- retrait de la réalité ;
- modèles de la pensée illogiques et/ou désorganisés ;
- croyances paranoïdes ou délirantes ;
- paranoïa ;
- folie des grandeurs ;
- délire de persécution ou d'auto-réprimande ; et
- changements de la personnalité.

**6.** Une 3,11b-*cis*-dihydrotétrabénazine à utiliser selon l'une quelconque des revendications 1 à 3, dans laquelle les épisodes psychotiques, les psychoses ou les symptômes prévenus ou soulagés sont sélectionnés parmi ceux découlant de ou associés à :

- une psychose causée par ou associée à la schizophrénie ;
- une psychose causée par ou associée au trouble bipolaire (dépression maniaque) ;
- une psychose causée par ou associée à une dépression clinique sévère ;
- une psychose induite par des troubles et affections telles que :

  ◊ trouble électrolytique :
  ◊ infections des voies urinaires chez les personnes âgées ;
  ◊ syndromes douloureux ;
  ◊ intoxication par un médicament ;
  ◊ sevrage thérapeutique ; et
  ◊ infections ou blessures du cerveau ;

- une psychose causée par un stress psychologique chronique (brève psychose réactive)
- une psychose déclenchée ou exacerbée par un stress mental sévère ; et
- une psychose déclenchée par ou découlant de ou succédant à des maladies ou affections telles que le SIDA, la lèpre, le paludisme et les oreillons.

**7.** Une 3,11b-*cis*-dihydrotétrabénazine à utiliser selon la revendication 4, dans laquelle l'usage est le soulagement ou la réduction d'un ou de plusieurs symptômes de la schizophrénie.

**8.** Une 3,11b-*cis*-dihydrotétrabénazine à utiliser selon la revendication 4, dans laquelle l'usage est la prévention, le soulagement ou la réduction d'un ou de plusieurs symptômes sélectionnés parmi :

- délire ;
- hallucinations ;
- confusion ;
- troubles émotionnels, comportementaux ou intellectuels ;
- retrait de la réalité ; et
- modèles illogiques de la pensée.

**9.** La 3,11b-*cis*-dihydrotétrabénazine à utiliser selon l'une quelconque des revendications précédentes, dans laquelle la 3,11b-*cis*-dihydrotétrabénazine se présente sous la forme d'un sel d'addition acide.

**10.** La 3,11b-*cis*-dihydrotétrabénazine à utiliser selon la revendication 9, dans laquelle le sel est un sulfonate de méthane.

**11.** La 3,11b-*cis*-dihydrotétrabénazine à utiliser selon la revendication 1, dans laquelle l'usage est l'amélioration des symptômes du déficit cognitif de la schizophrénie.

**12.** L'usage d'une 3,11b-*cis*-dihydrotétrabénazine ayant pour formule (Ib), telle que définie dans l'une quelconque des revendications 1, 9 et 10, pour la fabrication d'un médicament pour usage thérapeutique, tel que défini dans l'une quelconque des revendications 1 à 8 et 11.

# FIGURE 1

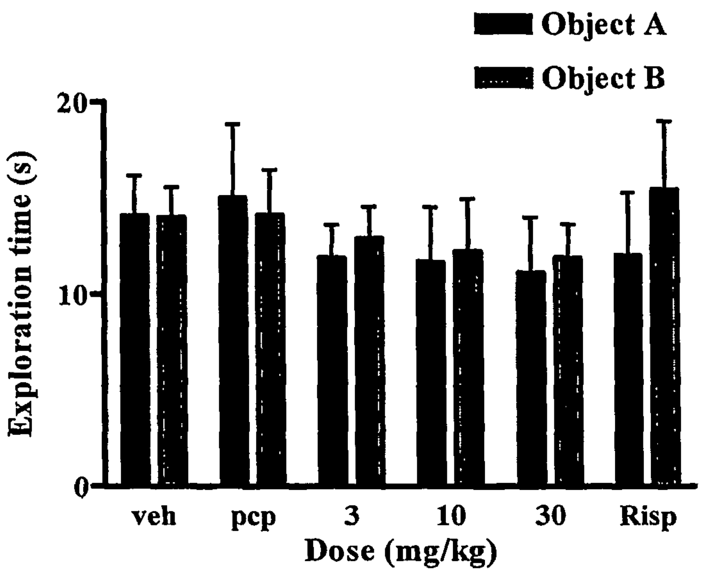


# FIGURE 2

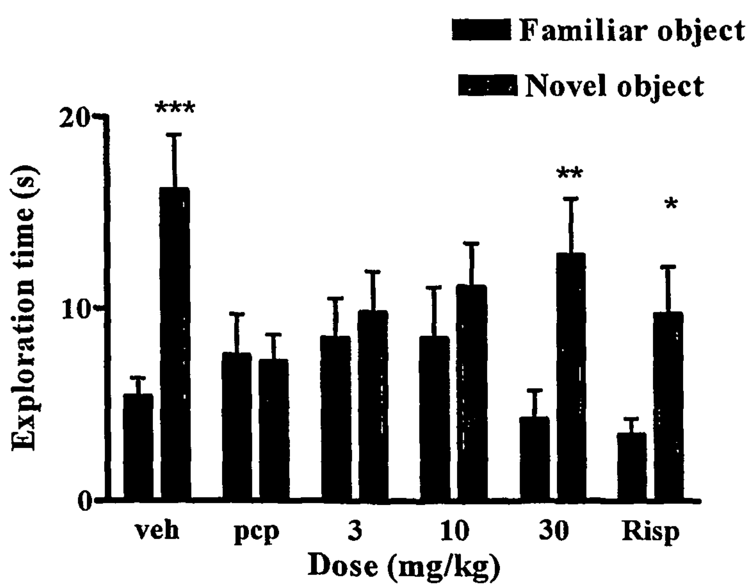

# FIGURE 3

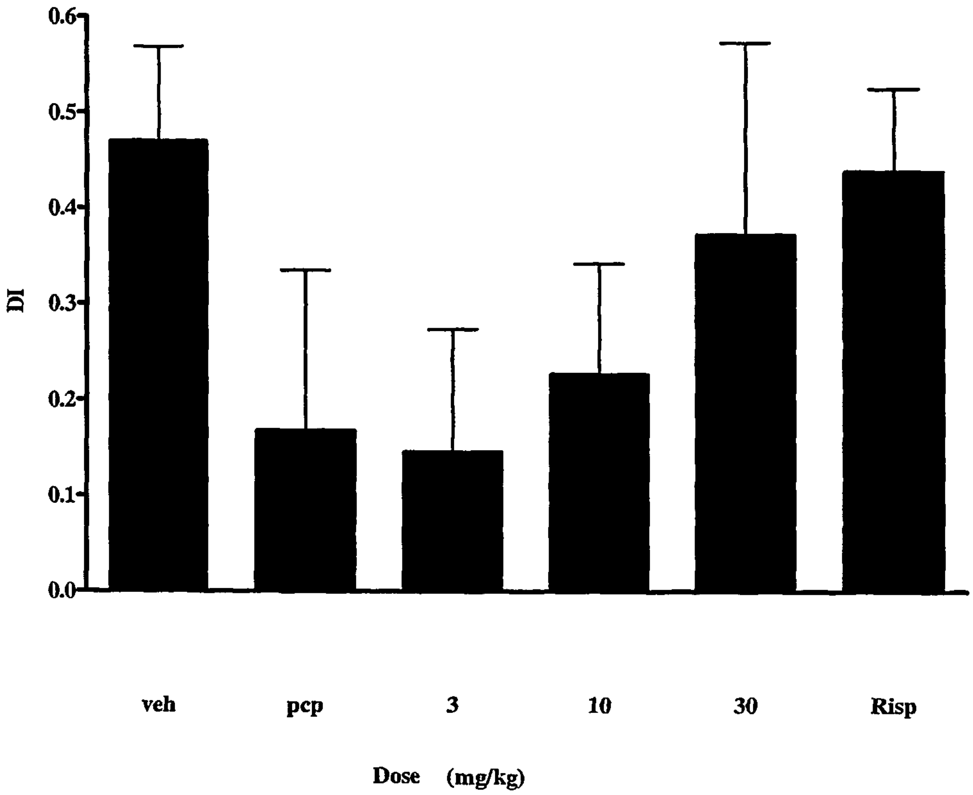

# FIGURE 4

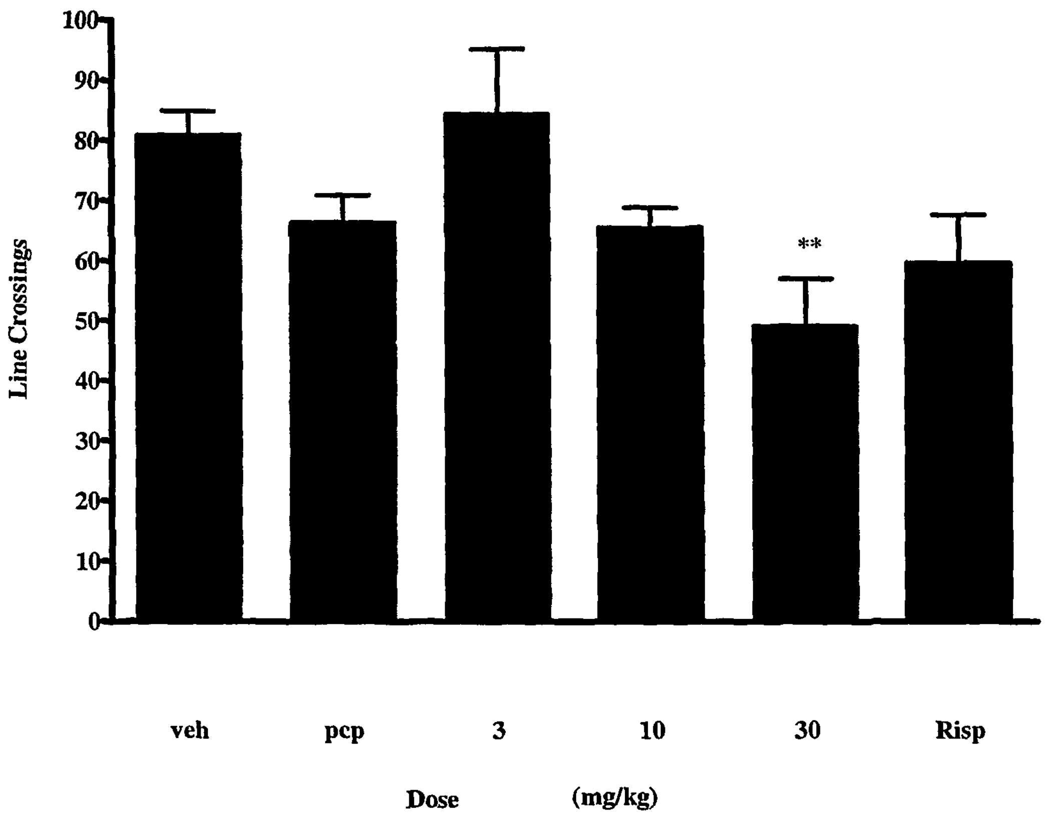
100
90
80
70
60
50
40
30
20
10
0
Line Crossings
**
veh
pcp
3
10
30
Risp
Dose
(mg/kg)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2005000464 W
- US 2843591 A, Hoffmann- La Roche
- US 2830993 A, Hoffmann-La Roche

### Non-patent literature cited in the description


- **A. E. HENSIEK ; M. R. TRIMBLE.** *J Neurology, Neurosurgery and Psychiatry,* 2002, vol. 72, 281-285
- **H. Y. MELTZER.** *J. Clin. Psychopharmacol.,* February 1995, vol. 15 (1 Suppl 1), 2S-3S
- **M. HUTTUNEN.** *J. Clin. Psychopharmacol.,* February 1995, vol. 15 (1 Suppl 1), 4S-10S
- **JANKOVIC et al.** *Am. J Psychiatry,* August 1999, vol. 156 (8), 1279-81
- **JANKOVIC et al.** *Neurology,* February 1997, vol. 48 (2), 358-62
- **JERRY MARCH.** Advanced Organic Chemistry. John Wiley & Sons, 1992, 109-114
- **MEHVAR et al.** *Drug Metab.Disp,* 1987, vol. 15, 250-255
- *J Pharm. Sci.,* 1987, vol. 76 (6), 461-465
- **KILBOURN et al.** *Chirality,* 1997, vol. 9, 59-62
- **BROSSI et al.** *Helv. Chim. Acta,* 1958, vol. XLI (193), 1793-1806
- **KILBOURN et al.** *Eur. J Pharmacol.,* 1995, vol. 278, 249-252
- *Med. Chem. Res.,* 1994, vol. 5, 113-126
- Pharmaceutical Salts: Properties, Selection, and Use. August 2002, 388
- Remington's Pharmaceutical Sciences. Mack Publishing Company
- **DUISENBERG, A.J.M.** *J Appl. Cryst.,* 1992, vol. 25, 92-96
- **Z. OTWINOWSKI ; W. MINOR.** Methods in Enzymology. *Macromolecular Crystallography, part A,* 1997, vol. 276, 307-326
- **G. M. SHELDRICK.** *Acta Cryst.,* 1990, vol. A46, 467-473
- **D. M. WATKIN ; L. PEARCE ; C. K. PROUT.** *Chemical Crystallography Laboratory,* 1993
- **S. UHLEN et al.** *J Pharmacol. Exp. Ther.,* 1994, vol. 271, 1558-1565
- **S. UHLEN et al.** *Eur. J. Pharmacol.,* 1998, vol. 33 (1), 93-11
- **DEARRY et al.** *Nature,* 1990, vol. 347, 72-76
- **BUNZO et al.** *Nature,* 1988, vol. 336, 783-787
- **SOKOLOFF et al.** *Nature,* 1990, vol. 347, 146-151
- **BROWN et al.** *Brit. J Pharmacol.,* 1990, vol. 99, 803-809
- **GANAPATHY et al.** *Pharmacol. Exp. Ther.,* 1999, vol. 289, 251-260
- **HASHIMOTO et al.** *Eur. J Pharmacol.,* 1993, vol. 236, 159-163
- **GU et al.** *J. Biol. Chem.,* 1994, vol. 269 (10), 7124-7130
- **GIROS et al.** *Trends Pharmacol. Sci.,* 1993, vol. 14, 43-49
- **GU et al.** *J. Biol. Chem.,* 1994, vol. 269, 7124-7130
- **UHLEN et al.** *J. Pharmacol. Exp. Ther.,* 1994, vol. 271, 1558-1565
- **BONHAUS et al.** *Br. J Pharmacol.,* 1995, vol. 115, 622-628
- **MONSMA et al.** *Mol. Pharmacol.,* 1993, vol. 43, 320-327
- **J. D. JENTSCH ; R. H. ROTH.** *Neuropsychopharmacology,* 1999, vol. 20 (3), 201-225
- **APARICIO-LEGARZA et al.** *Neurosci. Lett.,* 1997, vol. 232, 13-16
- **GEYER et al.** *Brain Res. Bull.,* 1990, vol. 25, 485-498
- **ENNACEUR ; DELACOUR.** *Behav. Brain Res.,* 1988, vol. 31, 47-59
- **SUTCLIFFE et al.** A preliminary investigation into the effects of gender on cognition in male and female rats using the novel object recognition paradigm. *Presented at the 96th meeting of the Society for Endocrinology,* 07 November 2005
- **GRAYSON ; NEILL.** *J. Psychopharmacology,* 2004, vol. 18, A55, http://wwwpA2online.org/vol12issue4-abst077P.html.
- **IDRIS et al.** *Soc. Neurosci. abstr.,* 2005, vol. 67, 15